(19) Europäisches Patentamt / European Patent Office / Office européen des brevets

(11) **EP 3 305 320 B1**

(12) **EUROPEAN PATENT SPECIFICATION**

(45) Date of publication and mention
of the grant of the patent:
**07.02.2024  Bulletin 2024/06**

(21) Application number: **16803120.1**

(22) Date of filing: **23.05.2016**

(51) International Patent Classification (IPC):
**A61K 39/39** (2006.01)     **A61K 39/00** (2006.01)
**A61K 47/02** (2006.01)     **A61K 47/14** (2017.01)
**A61K 47/22** (2006.01)     **A61K 47/24** (2006.01)
**A61K 47/28** (2006.01)     **A61K 47/34** (2017.01)
**A61P 37/04** (2006.01)     **A61K 9/00** (2006.01)

(52) Cooperative Patent Classification (CPC):
**A61K 39/39; A61K 9/0019; A61K 39/00;
A61P 37/04**

(86) International application number:
**PCT/JP2016/065216**

(87) International publication number:
**WO 2016/194685 (08.12.2016 Gazette 2016/49)**

(54) **ALUMINUM-CONTAINING ADJUVANT COMPOSITION, AND VACCINE COMPOSITION CONTAINING SAME**

ALUMINIUMHALTIGE ADJUVANSZUSAMMENSETZUNG UND IMPFSTOFFZUSAMMENSETZUNG DAMIT

COMPOSITION D'ADJUVANT CONTENANT DE L'ALUMINIUM ET COMPOSITION DE VACCIN LA CONTENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.06.2015  JP 2015112519**

(43) Date of publication of application:
**11.04.2018  Bulletin 2018/15**

(73) Proprietor: **Terumo Kabushiki Kaisha
Tokyo 151-0072 (JP)**

(72) Inventor: **SAKAGUCHI, Naoki
Ashigarakami-gun
Kanagawa 259-0151 (JP)**

(74) Representative: **Casalonga
Casalonga & Partners
Bayerstraße 71/73
80335 München (DE)**

(56) References cited:
**WO-A1-2004/056389     WO-A1-2013/180253
JP-A- 2011 525 190     JP-A- 2012 532 200**

**US-A1- 2013 323 320**

- SHINOBU WATARAI ET AL: "Efficiency of pH-Sensitive Fusogenic Polymer-Modified Liposomes as a Vaccine Carrier", THE SCIENTIFIC WORLD JOURNAL, vol. 2013, 1 January 2013 (2013-01-01), pages 1-8, XP055333744, DOI: 10.1155/2013/903234
- EIJI YUBA ET AL.: 'Shinki pH Kanjusei Tantai o Mochiita Peptide Tanpakushitsu no Cytosol Delivery' ANNUAL MEETING OF THE JAPAN SOCIETY OF DRUG DELIVERY SYSTEM PROGRAM YOKOSHU vol. 30, July 2014, page 209, XP009507720
- YUBA E. ET AL.: 'Dextran derivative-based pH-sensitive liposomes for cancer immunotherapy.' BIOMATERIALS vol. 35, no. 9, 2014, ISSN 0142-9612 pages 3091 - 3101, XP028829008
- YOSHIZAKI Y. ET AL.: 'Potentiation of pH-sensitive polymer-modified liposomes with cationic lipid inclusion as antigen delivery carriers for cancer immunotherapy.' BIOMATERIALS vol. 35, no. 28, 2014, ISSN 0142-9612 pages 8186 - 8196, XP028861819

**(Cont. next page)**

- WATARAI S. ET AL.: 'Efficiency of pH-sensitive fusogenic polymer-modified liposomes as a vaccine carrier.' SCIENTIFIC WORLD JOURNAL vol. 2013, 2013, ISSN 2356-6140 pages 1 - 7, XP055333744
- EIJI YUBA: 'Antigen delivery using pH-sensitive polymer-modified liposomes and their application to cancer immunotherapy' MAJOR HISTOCOMPATIBILITY COMPLEX (MHC vol. 20, no. 3, 2013, ISSN 2186-9995 pages 181 - 189, XP055333747
- LEE K.Y. ET AL.: 'Investigation of antigen delivery route in vivo and immune-boosting effects mediated by pH-sensitive liposomes encapsulated with K(b)-restricted CTL epitope.' BIOCHEMICAL AND BIOPHYSICAL RESEARCH COMMUNICATIONS vol. 292, no. 3, 2002, ISSN 0006-291X pages 682 - 688, XP055333749
- RICHARDS R.L. ET AL.: 'Liposomes, lipid A, and aluminum hydroxide enhance the immune response to a synthetic malaria sporozoite antigen.' INFECTION AND IMMUNITY vol. 56, no. 3, 1988, ISSN 0019-9567 pages 682 - 686, XP008034645

**Description**

Technical Field

The present invention relates to an adjuvant composition containing aluminum and a vaccine composition containing the same.

Background Art

An immune system is a protective mechanism for protecting a living body from diseases or the like by excluding foreign matters, abnormal cells, etc. invading the living body. In addition, an adjuvant is a substance used for enhancing the effect of a vaccine.

In general, the function of an immune system is expressed through two kinds of mechanisms, namely, humoral immunity and cell-mediated immunity.

The humoral immunity is a mechanism centered on a component present in the state of being dissolved in serum, such as an antibody and a complement. Induction of an antibody is important from the viewpoint of vaccine, and it is normally expressed in the following manner. An exogenous antigen such as bacteria which has invaded a living body is embraced into antigen presenting cells by endocytosis, and it is digested and decomposed into peptide fragments by protease in endosomes in the antigen presenting cells. The peptide fragments combine with major histocompatibility complex (MHC) class II molecules. The resulting complex is presented to CD4 positive T cells on the surfaces of the antigen presenting cells, so that the CD4 positive T cells are activated. Then, the activated CD4 positive T cells perform release of cytokine or the like, and, eventually, an antibody is produced from B cells. Note that the aforesaid MHC class II molecules express themselves in macrophages, dendritic cells, activated T cells, B cells, and the like. There are many adjuvants for enhancing an antigen which are officially approved. Among others, aluminum-containing substances have a long history, and have been widely used in the world. Examples of them include aluminum hydroxide, aluminum phosphate, aluminum potassium sulfate (alum), and aluminum chloride, which are also generically called alum.

On the other hand, the cell-mediated immunity is a mechanism centered on cells in charge of exclusion of foreign matters, such as macrophages, cytotoxic T lymphocytes (CTL), and natural killer cells. Induction of cell-mediated immunity has been attracting great attention, from the viewpoint of immunotherapy, because of its ability to exclude virus-infected cells and cancer cells. Particularly, realization of induction of CTL is expected, from the viewpoint of application to immunotherapy of cancers. The induction of CTL is normally expressed as follows. An endogenous antigen, such as a protein, which has been generated in virus-infected cells or cancer cells, is ubiquitinated and then decomposed into peptide by proteasome. The peptide resulting from the decomposition combines with MHC class I molecules. The resulting complex is presented to CD8 positive T cells on the surfaces of the antigen presenting cells, so that the CD8 positive T cells are activated. Then, the activated CD8 positive T cells differentiate into cytotoxic T lymphocytes (CTL). Note that the aforesaid MHC class I molecules exist on the cell surfaces of almost all nucleated cells and blood platelets.

In recent years, a lot of investigations have been being made on the so-called cross-presentation, which positively induces CTL by use of exogenous antigens. To be more specific, the exogenous antigen is decomposed in endosomes in the antigen presenting cells and then combines with MHC class II molecules, as mentioned above. However, in the cross-presentation, by making even a part of exogenous antigen pass through the cell membrane or endosome membrane so that it migrates to the cytoplasmic substrate, the exogenous antigen which has thus migrated to the cytoplasmic substrate functions like an endogenous antigen, finally combining with MHC class I molecules, to induce CTL.

In the cross-presentation, it is necessary for the exogenous antigen to pass through the endosome membrane, and this is investigated extensively. For example, Bioconjug. Chem. 2009 Feb. 20(2): 241-248 states that pH-sensitive poly (propylacrylic acid) (PPAA) conjugate efficiently transports an antigen to the cytoplasmic substrate. In addition, Immune Netw. 2013 Oct 13(5) 177-183 describes a vaccine delivery based on nano-particles for cancer immunotherapy.

As an adjuvant for enhancing cell-mediated immunity, at present, there is no one that is officially approved, and attempts are being made on search for a substance or material, novel synthesis, utilization of a virus component, and the like. It has been reported that adjuvants for enhancing antibodies, such as aluminum-containing substances, regrettably do not have an effect to enhance cell-mediated immunity (Immunology and Cell Biology 2004 82, 497-505).

Summary of Invention

There is a strong desire to realize induction of cell-mediated immunity such as CTL, from the viewpoint of developing vaccines for therapy, and investigations for search of adjuvant molecules and promotion of cross-presentation are being made vigorously. However, the effects achieved so far to realize the induction are not sufficient.

In addition, since most of the investigations use novel synthetic materials or virus components, there exists fear from the viewpoint of safety.

Accordingly, it is an object of the present invention to provide an adjuvant which is highly safe and effectively induces cell-mediated immunity through cross-presentation.

The present inventor made extensive and intensive researches, which resulted in a finding that the aforesaid object can be achieved by using a pH-sensitive carrier jointly with an aluminum-containing substance as a substance having stimuli that activate natural immunity (hereinafter sometimes referred also to simply as "natural immunity-activating substance"). Based on the finding, the present invention has been made.

Specifically, the aforesaid object of the present invention is achieved by the following means.

(1) An adjuvant composition including:

a pH-sensitive carrier containing:

at least one pH-sensitive compound selected from the group consisting of deoxycholic acid, cholic acid, ursodeoxycholic acid, chenodeoxycholic acid, hyodeoxycholic acid, glycodeoxycholic acid, glycyrrhizinic acid, glycyrrhetinic acid, and salts thereof, and
at least one amphipathic substance selected from the group consisting of phosphatidylcholine having only an acyl group having 10 to 12 carbon atoms, polyoxyethylene sorbitan monofatty acid ester having an acyl group having 12 to 18 carbon atoms, sorbitan fatty acid ester having only an acyl group having 16 to 18 carbon atoms, glycerol monooleate, glycerol dilaurate, glycerol distearate, glycerol dioleate, polyoxyethylene castor oil, and $\alpha$-tocopherol; and

an aluminum-containing substance, wherein the aluminum-containing substance is contained in an amount of 1 to 1,000 $\mu$g, as aluminum metal, per 100 nmol of the amphipathic substance;

(2) The adjuvant composition as described in (1), in which the aluminum-containing substance is at least one selected from the group consisting of aluminum hydroxide and aluminum phosphate;

(3) The adjuvant composition as described in (1) or (2),

in which the pH-sensitive carrier contains

at least one pH-sensitive compound selected from the group consisting of deoxycholic acid, cholic acid, ursodeoxycholic acid, chenodeoxycholic acid, hyodeoxycholic acid, glycodeoxycholic acid, glycyrrhizinic acid, glycyrrhetinic acid, and salts thereof, and
at least one amphipathic substance selected from the group consisting of phosphatidylcholine having only an acyl group having 10 to 12 carbon atoms, polyoxyethylene sorbitan monofatty acid ester having an acyl group having 12 to 18 carbon atoms, sorbitan fatty acid ester having only an acyl group having 16 to 18 carbon atoms, glycerol monooleate, glycerol dilaurate, glycerol distearate, glycerol dioleate, polyoxyethylene castor oil, and $\alpha$-tocopherol, and

the pH-sensitive carrier shows an effect of promoting a membrane-disruption function;

(4) The adjuvant composition as described in (3), in which the aluminum-containing substance is contained in an amount of 1 to 1,000 $\mu$g, as aluminum metal, per 100 nmol of the amphipathic substance;
(5) The adjuvant composition as described in (3) or (4), in which the pH-sensitive compound is contained in a proportion of not less than 10 mol per 100 mol of the amphipathic substance;
(6) A vaccine composition including:

the adjuvant composition as described in (1) or (2) and;
an antigen;

(7) A vaccine composition including:

the adjuvant composition as described in any one of (3) to (5); and
an antigen;

(8) The vaccine composition as described in (7), in which a content of the antigen is 3.2 to 400 $\mu$g per 100 nmol of the amphipathic substance;

(9) The vaccine composition as described in any one of (6) to (8), in which the antigen is a peptide or a protein;

(10) A method of producing an adjuvant composition, including a step of associating

at least one pH-sensitive compound selected from the group consisting of deoxycholic acid, cholic acid, ursodeoxycholic acid, chenodeoxycholic acid, hyodeoxycholic acid, glycodeoxycholic acid, glycyrrhizinic acid, glycyrrhetinic acid, and salts thereof,

at least one amphipathic substance selected from the group consisting of phosphatidylcholine having only an acyl group having 10 to 12 carbon atoms, polyoxyethylene sorbitan monofatty acid ester having an acyl group having 12 to 18 carbon atoms, sorbitan fatty acid ester having only an acyl group having 16 to 18 carbon atoms, glycerol monooleate, glycerol dilaurate, glycerol distearate, glycerol dioleate, polyoxyethylene castor oil, and $\alpha$-tocopherol, and

an aluminum-containing substance with one another, wherein the aluminum-containing substance is contained in an amount of 1 to 1,000 $\mu$g, as aluminum metal, per 100 nmol of the amphipathic substance; and

(11) A method of producing a vaccine composition, including:

a step of associating

at least one pH-sensitive compound selected from the group consisting of deoxycholic acid, cholic acid, ursodeoxycholic acid, chenodeoxycholic acid, hyodeoxycholic acid, glycodeoxycholic acid, glycyrrhizinic acid, glycyrrhetinic acid, and salts thereof,

at least one amphipathic substance selected from the group consisting of phosphatidylcholine having only an acyl group having 10 to 12 carbon atoms, polyoxyethylene sorbitan monofatty acid ester having an acyl group having 12 to 18 carbon atoms, sorbitan fatty acid ester having only an acyl group having 16 to 18 carbon atoms, glycerol monooleate, glycerol dilaurate, glycerol distearate, glycerol dioleate, polyoxyethyl-ene castor oil, and $\alpha$-tocopherol, and

an aluminum-containing substance with one another wherein the aluminum-containing substance is contained in an amount of 1 to 1,000 $\mu$g, as aluminum metal, per 100 nmol of the amphipathic substance; and

a step of mixing an antigen with an associated body obtained by the association.

Brief Description of Drawings

[FIG. 1]

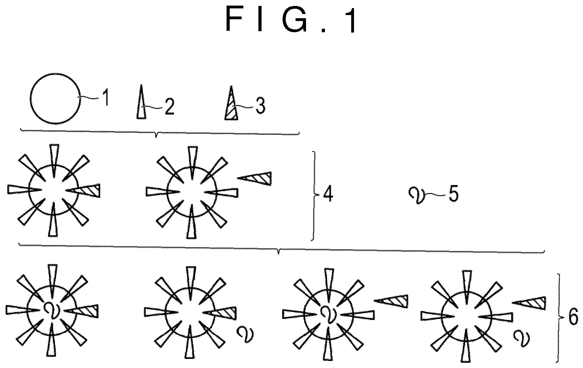

FIG. 1 is a schematic diagram of an adjuvant composition and a vaccine composition including the same according to an embodiment of the present invention.

[FIG. 2]

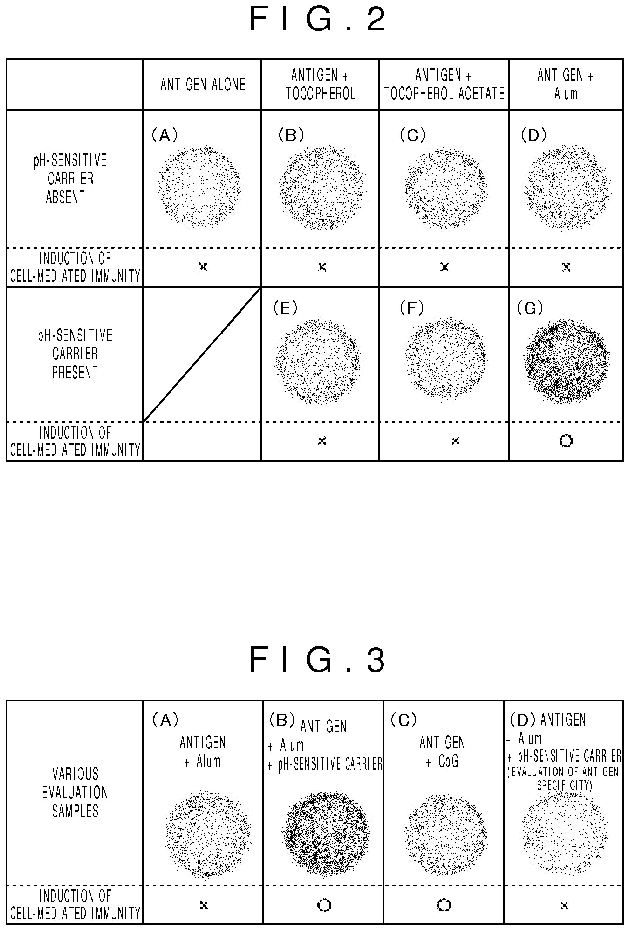

FIG. 2 is a diagram depicting the results of evaluation of induction of cell-mediated immunity (CTL) for vaccine compositions and comparative samples (compositions) by an ELIspot method. Cells that reacted with SIINFEKL, which is an MHC class I binding peptide, to produce IFN$\gamma$ were detected by formation of spot. The results are of the cases where immunization was conducted with a composition obtained by using (A) an antigen alone, (B) mixture of an antigen and tocopherol, (C) mixture of an antigen and tocopherol acetate, (D) mixture of an antigen and alum, (E) mixture of an antigen, tocopherol, and a pH-sensitive carrier, (F) mixture of an antigen, tocopherol acetate, and a pH-sensitive carrier, and (G) mixture of an antigen, alum, and a pH-sensitive carrier. Aluminum hydroxide was used as the alum, and a complex of DLPC and deoxycholic acid was used as the pH-sensitive carrier.

[FIG. 3]
FIG. 3 is a diagram depicting the results of comparison with CpG-ODN and evaluation of antigen specificity of cell-mediated immunity induced, by an ELIspot method. The results of the cases where immunization was conducted with a composition obtained by using (A) a mixture of an antigen and alum, (B) a mixture of an antigen, alum, and a pH-sensitive carrier, and (C) a mixture of an antigen and CpG-ODN. The result of (D) is a result of immunizing a mouse with a composition obtained by a mixture of an antigen, alum, and a pH-sensitive carrier and attempting spot formation without adding a peptide of SIINFEKL. Aluminum hydroxide was used as the alum, and a complex of DLPC and deoxycholic acid was used as the pH-sensitive carrier.

[FIG. 4]

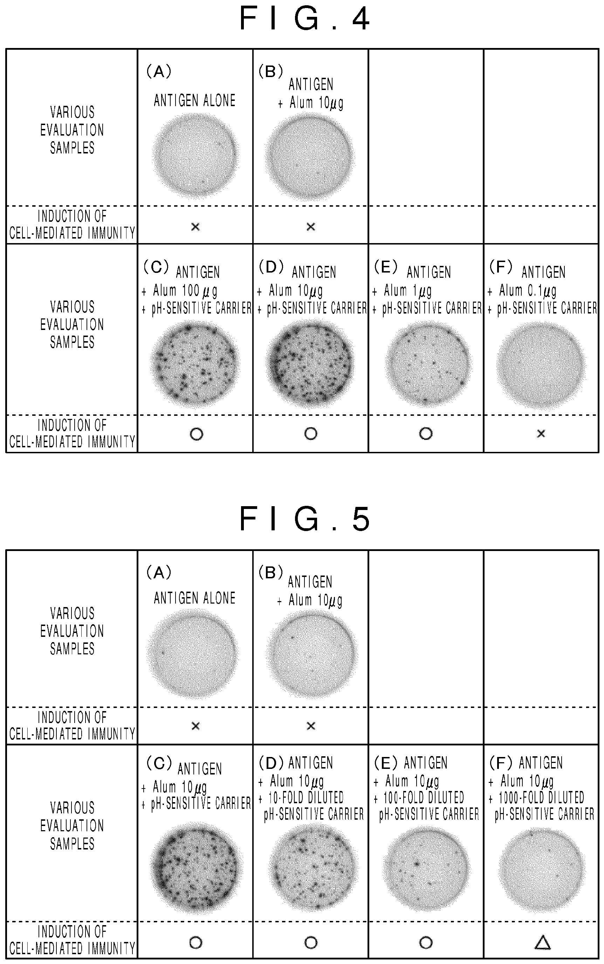

FIG. 4 is a diagram depicting the results of examination of the amount of alum needed for inducing cell-mediated immunity, by an ELIspot method. In each evaluation, a complex of 100 nmol of DLPC and 160 nmol of deoxycholic acid was used as the pH-sensitive carrier, and aluminum hydroxide was used as the alum. The results are of the cases where immunization was conducted with a composition obtained by (A) an antigen alone, (B) a mixture of an antigen and 10 μg of the alum, (C) a mixture of an antigen, 100 μg of the alum, and the pH-sensitive carrier, (D) a mixture of an antigen, 10 μg of the alum, and the pH-sensitive carrier, (E) a mixture of an antigen, 1 μg of the alum, and the pH-sensitive carrier, and (F) a mixture of an antigen, 0.1 μg of the alum, and the pH-sensitive carrier.

[FIG. 5]
FIG. 5 is a diagram depicting the results of examination of the amount of a pH-sensitive carrier needed for inducing cell-mediated immunity, by an ELIspot method. As the pH-sensitive carrier, a complex of 100 nmol of DLPC and 160 nmol of deoxycholic acid was used as a reference, and liquids obtained by diluting the complex into predetermined concentrations were each used for evaluation. Aluminum hydroxide was used as the alum. The results are of the cases where immunization was conducted with a composition obtained by using (A) an antigen alone, (B) a mixture of an antigen and the alum, (C) a mixture of an antigen, the alum, and the pH-sensitive carrier, (D) a mixture of an antigen, the alum, and a 10-fold diluted pH-sensitive carrier, (E) a mixture of an antigen, the alum, and a 100-fold diluted pH-sensitive carrier, and (F) an antigen, the alum, and a 1,000-fold diluted pH-sensitive carrier.

[FIG. 6]

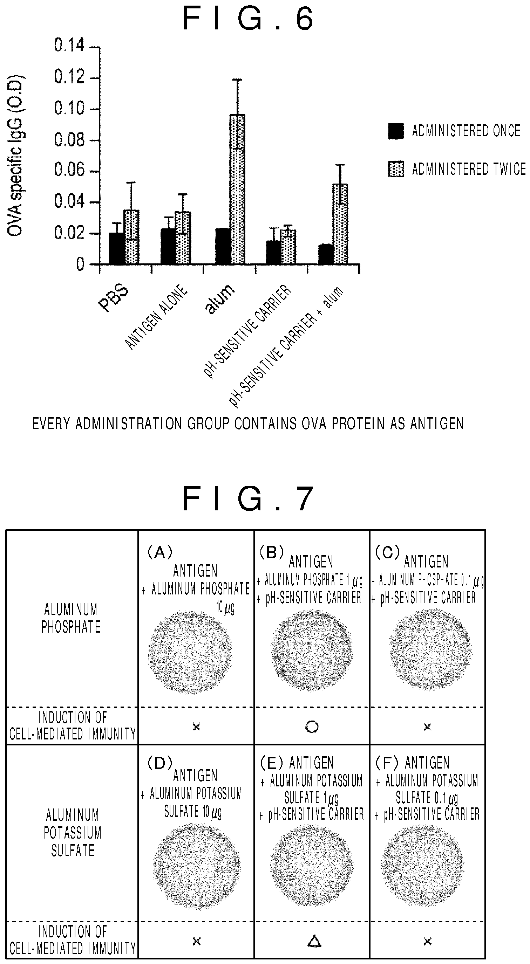

FIG. 6 is a diagram depicting the results of verification of induction of humoral immunity by joint use of an alum and a pH-sensitive carrier. The induction of humoral immunity was evaluated based on IgG antibody titer.

[FIG. 7]
FIG. 7 is a diagram depicting the results of examination of the kind of alum suitable as a constituent of a vaccine composition for inducing cell-mediated immunity, by an ELIspot method. In each evaluation, a complex of 100 nmol of DLPC and 160 nmol of deoxycholic acid was used as the pH-sensitive carrier, and aluminum hydride was used as the alum. The results are of the cases where immunization was conducted with a composition obtained by (A) a mixture of an antigen and 10 μg of aluminum phosphate, (B) a mixture of an antigen, 1 μg of aluminum phosphate, and the pH-sensitive carrier, (C) a mixture of an antigen, 0.1 μg of aluminum phosphate, and the pH-sensitive carrier, (D) a mixture of an antigen and 10 μg of aluminum potassium sulfate, (E) a mixture of an antigen, 1 μg of aluminum potassium sulfate, and the pH-sensitive carrier, and (F) a mixture of an antigen, 0.1 μg of aluminum potassium sulfate, and the pH-sensitive carrier.

[FIG. 8]

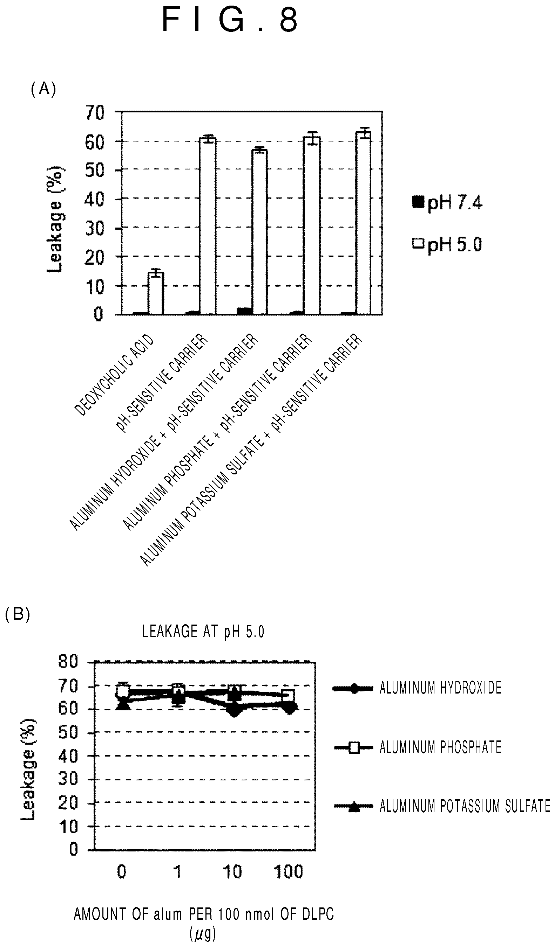

FIG. 8 is a diagram depicting the results of evaluation of the influence of alum on the pH-sensitive carrier's effect of promoting the membrane-disruption function, in the case of mixture of an alum and a pH-sensitive carrier. (A) depicts the results of evaluation of the kind of alum, and (B) depicts the results of evaluation of the amount of alum.

[FIG. 9]

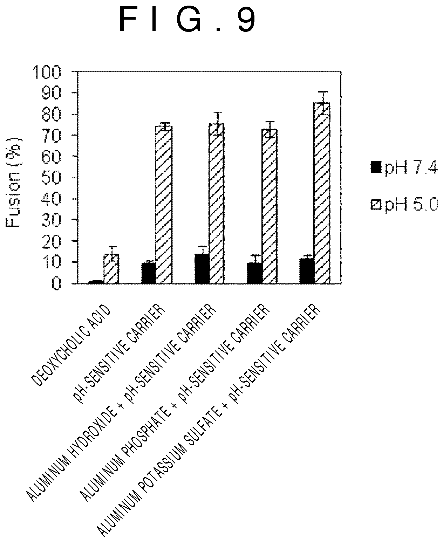

FIG. 9 is a diagram depicting the results of examination of the influence of alum on the pH-sensitive carrier's effect of promoting the membrane-disruption function.

[FIG. 10]

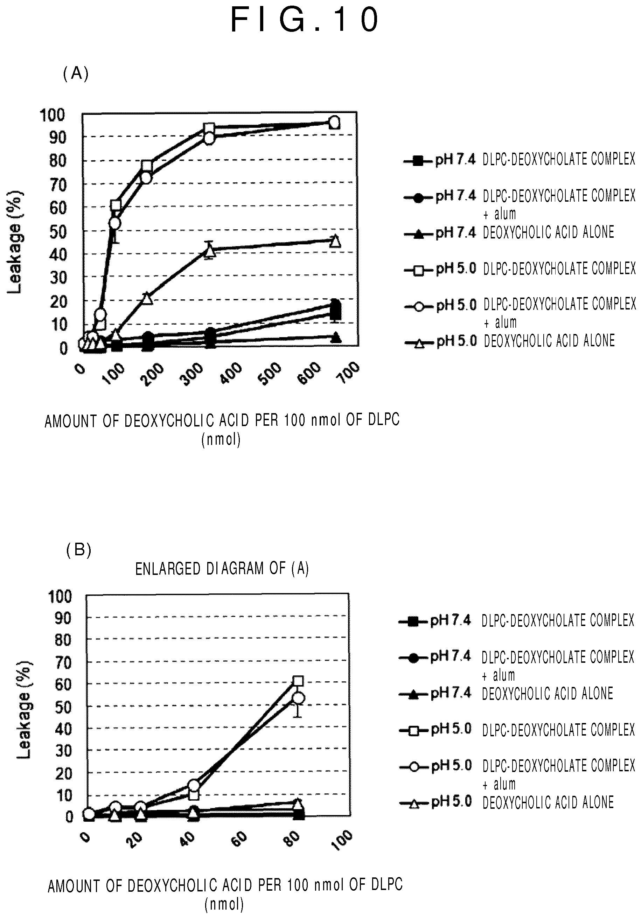

FIG. 10 is a diagram depicting the results of evaluation of the influence of alum on the pH-sensitive carrier's effect of promoting the membrane-disruption function, in the case where the composition of the pH-sensitive carrier, particularly, the amount of deoxycholic acid was varied. (A) depicts the results of examination for a range in which the amount of deoxycholic acid was 0 to 640 nmol, and (B) is an enlarged diagram for the range of 0 to 100 nmol.

[FIG. 11]

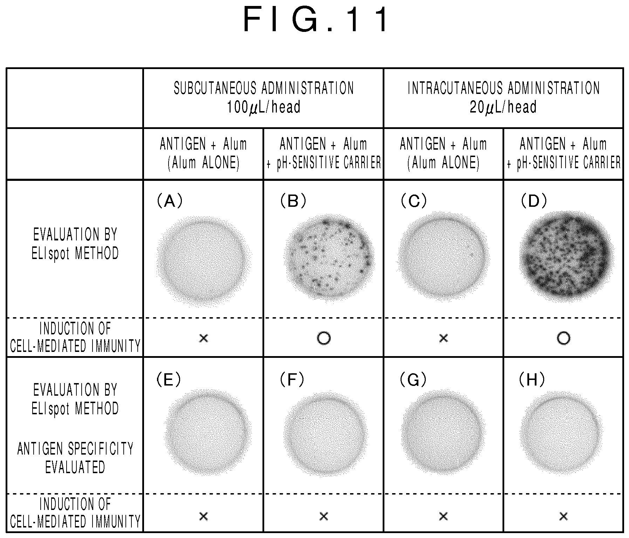

FIG. 11 is a diagram depicting the results of evaluation of induction of cell-mediated immunity by a vaccine composition, in an intracutaneous administration route, by an ELIspot method. In each evaluation, a complex of 100 nmol of DLPC and 160 nmol of deoxycholic acid was used as the pH-sensitive carrier, and aluminum hydroxide was used as the alum. (A) depicts the results of subcutaneous administration of a composition obtained by mixing an antigen and the alum, (B) depicts the results of subcutaneous administration of a composition obtained by mixing an antigen, the alum, and the pH-sensitive carrier, (C) depicts the results of intracutaneous administration of a composition obtained by mixing an antigen and the alum, (D) depicts the results of intracutaneous administration of a composition obtained by mixing an antigen, the alum, and the pH-sensitive carrier. Further, (E) to (H) depict the results of evaluation of antigen specificity, as an attempt to perform spot formation without adding a peptide of SIINFEKL. (E) depicts the results of subcutaneous administration of a composition obtained by mixing an antigen and the alum, (F) depicts the results of subcutaneous administration of a composition obtained by mixing an antigen, the alum and the pH-sensitive carrier, (G) depicts the results of intracutaneous administration of a composition obtained by mixing an antigen and the alum, and (H) depicts the results of intracutaneous administration of a composition obtained by mixing an antigen, the alum, and the pH-sensitive carrier.

Modes for Carrying Out the Invention

According to an aspect of the present invention, there is provided an adjuvant composition that includes a pH-sensitive carrier and an aluminum-containing substance. According to the aforesaid adjuvant composition, there is provided an adjuvant that can effectively induce cell-mediated immunity through cross-presentation. In addition, according to the adjuvant composition, there is provided an adjuvant that is high in safety.

<Adjuvant Composition>

The adjuvant composition according to the present aspect includes a pH-sensitive carrier (hereinafter sometimes referred to simply as "carrier," "associated body," or "complex") and an aluminum-containing substance. Specifically, the adjuvant composition of the present aspect includes an aluminum-containing substance as a substance having stimulus that activates natural immunity (a natural immunity-activating substance).

According to a preferred embodiment, the adjuvant composition of the present aspect includes a pH-sensitive carrier, an aluminum-containing substance, and an aqueous solvent. Therefore, the adjuvant composition according to the present aspect is preferably in a form in which a pH-sensitive carrier and an aluminum-containing substance are dispersed in an aqueous solvent.

In the adjuvant composition of the present aspect, the pH-sensitive carrier includes: at least one pH-sensitive compound selected from the group consisting of deoxycholic acid, cholic acid, ursodeoxycholic acid, chenodeoxycholic acid, hyodeoxycholic acid, glycodeoxycholic acid, glycyrrhizinic acid, glycyrrhetinic acid, and salts thereof; and at least one amphipathic substance selected from the group consisting of phosphatidylcholine having only an acyl group having 10 to 12 carbon atoms, polyoxyethylene sorbitan monofatty acid ester having an acyl group having 12 to 18 carbon atoms, sorbitan fatty acid ester having only an acyl group having 16 to 18 carbon atoms, glycerol monooleate, glycerol dilaurate, glycerol distearate, glycerol dioleate, polyoxyethylene castor oil, and $\alpha$-tocopherol, and produces an effect of promoting a membrane-disruption function.

While the present embodiment will be described below referring to the drawings, the technical scope of the present invention is to be defined based on the description in the claims and is not limited only to the following embodiments. Note that the dimensional ratios in the drawings are exaggerated for convenience of explanation, and may be different from the actual ratios.

Besides, herein operations and measurement of physical properties and the like are conducted under the conditions of a room temperature (20°C to 25°C) and a relative humidity of 40%RH to 50%RH, unless specified otherwise.

FIG. 1 is a schematic diagram of an adjuvant composition and a vaccine composition according to one embodiment of the present invention.

According to FIG. 1, an adjuvant composition 4 contains an amphipathic substance 1, a pH-sensitive compound 2, and an aluminum-containing substance 3, which is a substance having stimulus that activates natural immunity. As illustrated in FIG. 1, according to one embodiment, the aluminum-containing substance 3 associates with a hydrophobic moiety constituting the amphipathic substance 1, together with the pH-sensitive compound 2. In this case, the adjuvant composition 4 can be said to be an adjuvant complex. In addition, according to another embodiment, the aluminum-containing substance 3 exists independently from a pH-sensitive carrier that includes the amphipathic substance 1 and the pH-sensitive compound 2.

Besides, a vaccine composition 6 includes the adjuvant composition 4 and an antigen 5. As depicted in FIG. 1, the antigen 5 may be embraced in, or may exist independently from, the adjuvant composition 4 according to the aforesaid two embodiments. Among these, the vaccine composition 6 in which the antigen 5 is embraced in the adjuvant complex 4 can be also said to be a vaccine complex.

The "adjuvant composition" herein means a composition including a pH-sensitive carrier and an aluminum-containing substance, and its form is not specifically limited. In other words, the "adjuvant composition" may be a composition obtained by mixing a pH-sensitive carrier and an aluminum-containing substance, or may be a composition in which an aluminum-containing substance is supported on or embraced in a pH-sensitive carrier (adjuvant complex), and both of these types of compositions are herein collectively referred to as "adjuvant composition."

In addition, the "vaccine composition" herein means a composition including an adjuvant composition and an antigen, and its form is not particularly limited. In other words, the "vaccine composition" may be a composition in which two or more selected from the group consisting of the constituents of an adjuvant composition and an antigen are mixed together, or may be a composition in which an antigen is supported on or embraced in an adjuvant complex (vaccine complex), and both of these types of compositions are herein collectively referred to as "vaccine composition."

[pH-sensitive carrier]

The pH-sensitive carrier is sensitive to pH and has a function of being able to transport an antigen in cells to a cytoplasmic substrate when pH is brought into an acidic range.

While the pH-sensitive carrier is not particularly limited, it is preferably one which includes at least one pH-sensitive compound selected from the group consisting of deoxycholic acid, cholic acid, ursodeoxycholic acid, chenodeoxycholic acid, hyodeoxycholic acid, glycodeoxycholic acid, glycyrrhizinic acid, glycyrrhetinic acid, and salts thereof, and at least one amphipathic substance selected from the group consisting of phosphatidylcholine having only an acyl group having 10 to 12 carbon atoms, polyoxyethylene sorbitan monofatty acid ester having an acyl group having 12 to 18 carbon atoms, sorbitan fatty acid ester having only an acyl group having 16 to 18 carbon atoms, glycerol monooleate, glycerol dilaurate, glycerol distearate, glycerol dioleate, polyoxyethylene castor oil, and $\alpha$-tocopherol, and which shows an effect of promoting a membrane-disruption function. Note that the "carbon number" used herein for the amphipathic substance means the number of carbon atoms in the fatty acid component (acyl group) constituting the hydrophobic moiety of the amphipathic substance.

The pH-sensitive carrier which includes the pH-sensitive compound and the amphipathic substance and which shows an effect of promoting a membrane-disruption function will be described in detail below.

Note that, as the pH-sensitive carrier according to the present aspect, the "pH-sensitive carrier" described in International Publication No. WO2013/180253 filed on May 24, 2013 can be used in the same manner.

(Structure of pH-sensitive carrier)

It is considered that the pH-sensitive carrier is formed by association between the pH-sensitive compound and the amphipathic substance at a physiological pH or above. To be more specific, it is considered that the pH-sensitive carrier is formed when the pH-sensitive compound associates with the hydrophobic moiety of the amphipathic substance. Note that the form of association of the pH-sensitive carrier is an inferred one, and pH-sensitive carrier is not limited by the form of association.

(Effect of promoting membrane-disruption function)

The term "membrane-disruption function" means a function to bring about release in the release test. The release test described herein is a test consisting of the steps of adding liposomes (dispersion) embracing an aqueous solution containing a quenching substance and a fluorescent substance and a dispersion of an evaluation sample to a properly pH-adjusted aqueous solution, incubating the resulting aqueous solution at 37°C for 90 minutes or 30 minutes, and measuring the intensity of fluorescence of the incubated aqueous solution. This method makes it possible to measure the amount of the fluorescent substance which has been released from the liposomes, and to confirm the membrane-disruption function of the pH-sensitive carrier. Note that the release test will be described in more detail in the Examples to be described later.

The term "to show the effect of promoting the membrane-disruption function" means that the following two conditions are satisfied simultaneously.

(1) The release test indicates that the leakage is higher at a predetermined pH (lower than the physiological pH) than that at the physiological pH, and the amount of increase is larger than that observed in the experiment which is carried out with the pH-sensitive compound alone.

(2) The release test at a predetermined pH lower than the physiological pH indicates that the leakage in the case where the pH-sensitive compound and the amphipathic substance form a complex (the pH-sensitive carrier) is larger than the sum of the leakage due to the pH-sensitive compound used alone and the leakage due to the amphipathic substance used alone. To be more specific, the term "to show the effect of promoting the membrane-disruption function" means the following: the release test at pH 7.4 and pH 5.0 or pH 4.5 indicates that the relation among La, Lb, and Lc exists as indicated by the following two formulas, where La represents the leakage for the pH-sensitive compound existing alone, Lb represents the leakage for the amphipathic substance existing alone, and Lc represents the leakage for the pH-sensitive carrier (which is a complex of the pH-sensitive compound and the amphipathic substance). In other words, what is mentioned in aforesaid (1) and (2) is expressed in terms of the formulas (1) and (2) below, respectively. In the following formulas, $Lc_{7.4}$, $La_{7.4}$, and $Lb_{7.4}$ represent the leakage at pH 7.4, and $Lc_x$, $La_x$, and $Lb_x$ represent the leakage at pH 5.0 or pH 4.5.

[Math. 1]

$$\text{Formula (1)} \quad \Delta = (Lc_x - Lc_{7.4}) - (La_x - La_{7.4}) > 0$$

$$\text{Formula (2)} \quad \Delta' = Lc_x - (La_x + Lb_x) > 0$$

In the aforesaid formula (1), it is sufficient that $\Delta$ is more than 0, but $\Delta$ is preferably not less than 2, more preferably not less than 2.5, further preferably not less than 5, particularly preferably not less than 10, and most preferably not less than 30. Besides, in the aforesaid formula (2), it is sufficient that $\Delta'$ is more than 0, but $\Delta'$ is preferably not less than 2, more preferably not less than 2.5, further preferably not less than 3, still further preferably not less than 5, particularly preferably not less than 10, and most preferably not less than 15.

In one embodiment of the present invention, it is preferable that both $\Delta$ and $\Delta'$ in the aforesaid formulas (1) and (2) are not less than 2 (preferably not less than 5), and that the pH-sensitive carrier contains a bile acid and a lipid. Besides, in another embodiment of the present invention, it is preferable that both $\Delta$ and $\Delta'$ in the aforesaid formulas (1) and (2) are not less than 2 (preferably not less than 5), and that the pH-sensitive carrier contains glycyrrhizinic acid or glycyrrhetinic acid and a lipid.

In this specification, the term "physiological pH" means a pH value in normal tissue or normal body fluid. The physiological pH is usually 7.4, but may slightly vary ($\pm$ 0.1) from one normal issue or one normal body fluid to another. The term "predetermined pH lower than the physiological pH" means a pH which is sufficient if it is lower than 7.4 and which is preferably a pH of not less than 3.0 and less than 7.4, more preferably a pH of not less than 4.0 and less than 7.3, and further preferably a pH of not less than 4.5 and less than 7.0.

No elucidation has been made yet on the mechanism by which the pH-sensitive carrier shows the effect of promoting the membrane-disruption function.

It is assumed that the pH-sensitive carrier changes the mode of association between the pH-sensitive compound and the amphipathic substance when the environment decreases in pH below the physiological pH, and this leads to the effect of promoting the membrane-disruption function. For example, when a system containing a pH-sensitive carrier and a biomembrane (such as cell membrane and vesicle membrane) changes such that its pH decreases below the physiological pH, the pH-sensitive carrier would change in the mode of association. Then, after the pH-sensitive carrier contacts with the biomembrane, change in the structure of the biomembrane is induced by the change. In other words, the pH-sensitive carrier would bring about change in the structure of the biomembrane. A probable reason for this is that a change of pH into a weak acid range makes unstable the pH-sensitive compound in the pH-sensitive carrier in the structure of the carrier, with the result that the pH-sensitive carrier undergoes rearrangement with the biomembrane existing in the system, thereby showing the effect of promoting the membrane-disruption function. The foregoing may be put in another way as follows. The pH-sensitive compound consists of molecules which, when pH is changed into a weak acid range, undergo protonation and change the state of hydrophobic association. In other words, the hydrophobic association involving the pH-sensitive compound can show its membrane disruption function by responding to the weak acid environment. Note that the term "membrane disruption" means such a change in membrane structure, and it does not necessarily mean that the membrane-constituting components are entirely separated or decomposed. As a result of such "membrane disruption," any component that can be contained inside the biomembrane (such as endosome) is, for example, released to the outside (e.g., cytoplasmic substrate) of the biomembrane.

The pH-sensitive carrier is preferably one which has a leakage lower than 20% at pH 7.4 and higher than 20% at pH 4.0 in the release test. The leakage in the release test is more preferably lower than 20% at pH 6.5 and higher than 20% at pH 4.0. The leakage at pH 7.4 or pH 6.5 is more preferably not more than 15%, and further preferably not more than 10%. The leakage at pH 4.0 is more preferably not less than 40%, and further preferably not less than 50%. In the adjuvant composition of the present mode, the leakage in the release test is preferably less than 20% at pH 7.4 and more than 20% at pH 5.0. With the leakage of the pH-sensitive carrier as defined above, the pH-sensitive carrier shows more conspicuously the effect of promoting the membrane-disruption function at a pH in a weak acid range. In the adjuvant composition of the present aspect, it is sufficient to use a pH-sensitive carrier having the effect of promoting the membrane-disruption function as aforementioned.

In addition, the pH-sensitive carrier shows not only the effect of promoting the membrane-disruption function but also the effect of promoting the membrane-fusion function.

The term "membrane-fusion function" as used in the present invention means the function that brings about membrane-fusion in the membrane-fusion test. The membrane-fusion test herein denotes a test which consists of the steps of adding liposomes (dispersion) having two kinds of fluorescent substances incorporated into the bimolecular membrane and an evaluation sample dispersion into an aqueous solution adjusted to a predetermined pH, incubating the resulting aqueous solution at 37°C for 60 minutes, and measuring the intensity of fluorescence of the incubated aqueous solution. This method makes it possible to measure change in energy resonance transfer for the two kinds of fluorescent substances incorporated into the liposomes and to confirm the membrane-fusion function of the pH-sensitive carrier. Note that a detailed description of the membrane-fusion test will be given later in Examples.

The term "to show the effect of promoting the membrane-fusion function" means a situation in which the membrane-fusion test indicates that the fusion rate at a predetermined pH lower than the physiological pH is higher than the fusion rate at the physiological pH and the amount of increase is larger than that observed when the pH-sensitive compound is tested alone. To be more specific, the term "to show the effect of promoting the membrane-fusion function" means that the membrane-fusion tests at pH 7.4 and pH 5.0 indicate that the relationship represented by the following formula (3) between the fusion rate Rc(%) of the pH-sensitive carrier (which is a complex of a pH-sensitive compound and an amphipathic substance) and the fusion rate Ra(%) of the pH-sensitive compound used alone is met. Note that, in the formula below, $Rc_{7.4}$ and $Ra_{7.4}$ represent the fusion rate at pH 7.4, respectively, and $Rc_x$ and $Ra_x$ represent the fusion rate at pH 5.0, respectively.

[Math. 2]

$$\text{Formula (3)} \quad \Delta R = (Rc_x - Rc_{7.4}) - (Ra_x - Ra_{7.4}) > 0$$

In the aforesaid formula (3), $\Delta R$ is sufficient if it is more than 0, but $\Delta R$ is preferably not less than 2, more preferably not less than 5, and further preferably not less than 10.

The pH-sensitive carrier is preferably one which has $\Delta R$ of not less than 2 in the aforesaid formula (3) and which contains a bile acid and a lipid.

The pH-sensitive carrier shows the effect of promoting the membrane-fusion function at a pH in a weak acid range (a predetermined pH lower than the physiological pH). Although the mechanism of this phenomenon is not yet elucidated, the mechanism is probably similar to that in the effect of promoting the membrane-disruption function mentioned above

That is, it is inferred that the pH-sensitive carrier used in the present invention contributes to membrane-fusion as a result of rearrangement with biomembranes existing in the system, through a change in the mode of association between the pH-sensitive compound and the amphipathic substance, in the case where the environment decreases in pH below the physiological pH. In this case, the membrane-fusion takes place as a result of rearrangement among components having affinity for each other, and hence those components which have no or little affinity for biomembrane (such as antigen) are excluded and released from the membrane which undergoes rearrangement.

As mentioned above, the antigen is usually surrounded by endosome, which is one kind of biomembrane, so that it is taken into cells (such as antigen presenting cells). After that, the endosome decreases in pH due to proton pumping action. Further, the endosome fuses with lysosome containing hydrolases, and the antigen is decomposed (Subsequently, the decomposed antigen forms a complex with MHC class II molecules, thereby being presented to CD4 positive T cells). Therefore, most of the antigen remains undelivered into the cytoplasmic substrate.

By contrast, the pH-sensitive carrier according to the present aspect permits the antigen (such as exogenous antigen) to be delivered to the cytoplasmic substrate. To be more specific, an environment with a decreased pH similarly occurs when the antigen together with the pH-sensitive carrier is surrounded by endosomes and taken into cells. As a result of the pH decrease (or acidification), the pH-sensitive compound makes unstable the pH-sensitive carrier, so that the rearrangement of membranes takes place between the endosome and the pH-sensitive carrier. This causes the pH-sensitive carrier to show the membrane-disruption function (which shows itself together with the membrane-fusion function in some cases). This membrane-disruption function (or both the membrane-fusion function and the membrane-disruption function) causes the antigen to be delivered into the cytoplasmic substrate from the endosome. Note that the aforesaid mechanism suggests that, in principle, the antigen can be transported to the cytoplasmic substrate once it has been taken into the endosome together with the pH-sensitive carrier. This indicates the possibility of using the antigen and the pH-sensitive carrier in the form of their mixed composition or using the antigen in such a form that it is supported on or embraced in the pH-sensitive carrier.


(pH-sensitive compound)

As mentioned above, the pH-sensitive compound is at least one selected from the group consisting of deoxycholic acid, cholic acid, ursodeoxycholic acid, chenodeoxycholic acid, hyodeoxycholic acid, glycodeoxycholic acid, glycyrrhizinic acid, glycyrrhetinic acid, and salts thereof. The aforesaid "salts thereof" are not specifically limited, and examples thereof include: alkali metal salts such as lithium salt, sodium salt, and potassium salt; alkaline earth metal salts such as magnesium salt, calcium salt, and barium salt; and ammonium salt. These pH-sensitive compounds may be used either singly or in combination of two or more of them.

According to one embodiment of the present invention, the pH-sensitive compound is more preferably at least one selected from the group consisting of deoxycholic acid, cholic acid, ursodeoxycholic acid, chenodeoxycholic acid, hyodeoxycholic acid, glycodeoxycholic acid, glycyrrhizinic acid, and salts thereof.

In addition, according to another embodiment of the present invention, the pH-sensitive compound is further preferably at least one selected from the group consisting of deoxycholic acid, ursodeoxycholic acid, chenodeoxycholic acid, hyodeoxycholic acid, glycodeoxycholic acid, glycyrrhizinic acid or salts thereof, and is particularly preferably at least one selected from the group consisting of deoxycholic acid, ursodeoxycholic acid, glycyrrhizinic acid or salts thereof.

Deoxycholic acid, cholic acid, ursodeoxycholic acid, chenodeoxycholic acid, hyodeoxycholic acid, and glycodeoxycholic acid, which are preferably used as the pH-sensitive compound, are generically called bile acids. The bile acids have been known as a typical steroid derivative for a long time before the 1920s and used in the field of bacteriology. The bile acids in the living human body form a complex with cholesterol, lipid, and fat-soluble vitamin, thereby assisting their absorption. Moreover, the bile acids form a complex with lipid, protein, or hydrophobic material owing to its physicochemical properties, and hence it has long been used for protein separation and purification and also as a solubilizer or emulsifier. Recently, it is attracting attention in the field of vaccine production because of its ability to promote drug absorption with the help of bile acid transporter. Typical examples of bile acids for such purposes are sodium deoxycholate (also known as sodium desoxycholate) and ursodeoxycholic acid (also known as ursodesoxycholic acid); they have found use as a pharmaceutical additive suitable for injection into human bodies owing to high safety. For this reason, the pH-sensitive compound is more preferably selected from deoxycholic acid, ursodeoxycholic acid, and salts thereof (such as sodium salt).

The pH-sensitive compound is preferably contained in a proportion of not less than 10 mol, more preferably in a proportion of 10 to 640 mol, further preferably in a proportion of 20 to 320 mol, and particularly preferably in a proportion of 20 to 160 mol, per 100 mol of the amphipathic substance. Note that the content of the pH-sensitive compound per 100 mol of the amphipathic substance is herein referred to also as "complexed amount of the pH-sensitive compound."

(Amphipathic substance)

As mentioned above, the amphipathic substance is preferably at least one selected from the group consisting of phosphatidylcholine having only an acyl group having 10 to 12 carbon atoms, polyoxyethylene sorbitan monofatty acid ester having an acyl group having 12 to 18 carbon atoms, sorbitan fatty acid ester having only an acyl group having 16 to 18 carbon atoms, glycerol monooleate, glycerol dilaurate, glycerol distearate, glycerol dioleate, polyoxyethylene castor oil, and α-tocopherol. These amphipathic substances may be used either singly or in combination of two or more of them.

Note that the "carbon number" used herein for the amphipathic substance means the number of carbon atoms in the fatty acid component (acyl group) constituting the hydrophobic moiety of the amphipathic substance.

Phosphatidylcholine having only an acyl group having 10 to 12 carbon atoms is preferably diacyl phosphatidylcholine having a saturated acyl group, and examples thereof include didecanoylphosphatidylcholine (DDPC; 1,2-didecanoyl-sn-glycero-3-phosphatidylcholine) and dilauroylphosphatidylcholine (DLPC; 1,2-dilauroyl-sn-glycero-3-phosphatidylcholine). Among these, as the phosphatidylcholine, natural one or synthetic one prepared by a known method may be used, and commercial one can be used.

Examples of the polyoxyethylene sorbitan monofatty acid ester having an acyl group having 12 to 18 carbon atoms include polyoxyethylene sorbitan monolauric acid ester (polyoxyethylene sorbitan monolaurate), polyoxyethylene sorbitan myristic acid ester (polyethylene sorbitan monomyristate), polyoxyethylene sorbitan monopalmitic acid ester (polyoxyethylene sorbitan palmitate), polyoxyethylene sorbitan monostearic acid ester (polyoxyethylene sorbitan monostearate), and polyoxyethylene sorbitan monooleic acid ester (polyoxyethylene sorbitan monooleate). Although the polymerization degree of polyoxyethylene is not particularly limited, the sum total of the polymerization degrees of respective polyoxyethylene chains added to sorbitan is preferably 10 to 200, more preferably 15 to 100, and further preferably 20 to 50. As the polyoxyethylene sorbitan monofatty acid ester, a synthetic one may be used or a commercial one may be used. As a commercial one of polyoxyethylene sorbitan monofatty acid ester, there can be used, for example, those commercialized under the trade names of Tween 20 (polyoxyethylene sorbitan monolauric acid ester), Tween 40 (polyoxyethylene sorbitan monopalmitic acid ester), Tween 60 (polyoxyethylene sorbitan monostearic acid ester), and Tween 80 (polyoxyethylene sorbitan monooleic acid ester). Among these, preferably used are polyoxyethylene sorbitan monofatty acid esters having 16 to 18 carbon atoms (Tween 20, Tween 40, Tween 60, and Tween 80).

Examples of sorbitan fatty acid ester having only an acyl group having 16 to 18 carbon atoms include: sorbitan monofatty acid esters such as sorbitan monopalmitic acid ester (sorbitan monopalmitate), sorbitan monostearic acid ester (sorbitan monostearate), and sorbitan monooleic acid ester (sorbitan monooleate); and sorbitan trifatty acid esters such as sorbitan tripalmitic acid ester (sorbitan tripalmitate), sorbitan tristearic acid ester (sorbitan tristearate), and sorbitan trioleic acid ester (sorbitan trioleate). As the sorbitan fatty acid ester, a synthetic one may be used or a commercial one may be used. As a commercial one of the sorbitan fatty acid ester, preferably usable are those commercialized under the trade names of SPAN 40 (sorbitan palimitic acid ester), SPAN 60 (sorbitan stearic acid ester), SPAN 80 (sorbitan oleic acid ester), SPAN 65 (sorbitan tristearic acid ester), and SPAN 85 (sorbitan trioleic acid ester). Among these, preferably used are SPAN 80, SPAN 65, and SPAN 85.

The glycerol monooleate (glyceryl monooleate), glycerol dilaurate (glyceryl dilaurate), glycerol distearate (glyceryl distearate), and glycerol dioleate (glyceryl dioleate) are acyl glycerols formed by ester linkage from glycerin and one or more molecules of fatty acid. The site of linkage for the fatty acid is not specifically limited. In the case of glycerol monooleate, which is a monoacyl glycerol, for example, the ester linkage of the fatty acid may exist at the C1 or C2 position of glycerin. Also, in the case of glycerol dilaurate, which is a diacyl glycerol, the ester linkage of the fatty acid may exist at the C1 and C2 positions of glycerin or at the C1 and C3 positions of glycerin. A preferable example of

glycerol dilaurate is $\alpha,\alpha'$-dilaurin, in which substitution occurs at the C1 and C3 positions. Preferable as glycerol distearate and glycerol dioleate are diacyl glycerols in which substitution occurs at the C1 and C2 positions. As these glycerol derivatives, synthetic ones may be used or commercial ones may be used.

The polyoxyethylene castor oil is formed by addition of polyoxyethylene to castor oil. The polymerization degree of the polyoxyethylene is not particularly limited, but it is preferably 3 to 200, more preferably 5 to 100, and further preferably 10 to 50. As the polyoxyethylene castor oil, a synthetic one may be used or a commercial one may be used. For example, PEG(10) castor oil, in which the polymerization degree of polyoxyethylene is 10, is suitably used.

As the $\alpha$-tocopherol, a natural one or a synthetic one prepared by a known method may be used, and a commercial one may also be used.

Among the aforementioned amphipathic substances, preferred are those selected from the group consisting of phosphatidylcholine having only an acyl group having 10 to 12 carbon atoms, polyoxyethylene sorbitan monolaurate, polyoxyethylene sorbitan monooleate, sorbitan monooleate, polyoxyethylene castor oil, and $\alpha$-tocopherol, and more preferred are those selected from the group consisting of dilauroyl phosphatidylcholine, didecanoyl phosphatidylcholine, polyoxyethylene sorbitan monolaurate, polyoxyethylene sorbitan monooleate, sorbitan monooleate, polyoxyethylene castor oil, and $\alpha$-tocopherol.

(Combination of pH-sensitive compound and amphipathic substance)

The pH-sensitive carrier can show the effect of promoting the membrane-disruption function at a desired pH owing to the combination of the pH-sensitive compound and the amphipathic substance. In this case, the pH at which the pH-sensitive carrier starts to show the effect of promoting the membrane-disruption function differs depending on the combination of the pH-sensitive compound and the amphipathic substance. This phenomenon is considered to arise from the fact that pKa varies from one pH-sensitive compound to another and the mode of association with the amphipathic substance also varies depending on the combination of the pH-sensitive compound and the amphipathic substance. Therefore, it can be said that is it possible, by appropriately changing the combination of the pH-sensitive compound and the amphipathic substance, to select the pH at which the function is shown and to set the delivery precisely.

In the pH-sensitive carrier, preferred as the combination of the pH-sensitive compound and the amphipathic substance are: cholic acid and DLPC; deoxycholic acid and DDPC; deoxycholic acid and DLPC; deoxycholic acid and Tween 20; deoxycholic acid and Tween 40; deoxycholic acid and Tween 60; deoxycholic acid and Tween 80; deoxycholic acid and SPAN 40; deoxycholic acid and SPAN 60; deoxycholic acid and SPAN 80; deoxycholic acid and SPAN 65; deoxycholic acid and SPAN 85; deoxycholic acid and $\alpha$-tocopherol; deoxycholic acid and glycerol monooleate; deoxycholic acid and glycerol distearate; deoxycholic acid and glycerol dioleate; deoxycholic acid and glycerol dilaurate ($\alpha,\alpha'$-dilaurin); deoxycholic acid and polyoxyethylene castor oil; chenodeoxycholic acid and DLPC; hyodeoxycholic acid and DLPC; glycodeoxycholic acid and DLPC; ursodeoxycholic acid and DDPC; ursodeoxycholic acid and DLPC; ursodeoxycholic acid and Tween 20; ursodeoxycholic acid and Tween 40; ursodeoxycholic acid and Tween 60; ursodeoxycholic acid and Tween 80; ursodeoxycholic acid and SPAN 40; ursodeoxycholic acid and SPAN 60; ursodeoxycholic acid and SPAN 80; ursodeoxycholic acid and SPAN 65; ursodeoxycholic acid and SPAN 85; ursodeoxycholic acid and $\alpha$-tocopherol; ursodeoxycholic acid and glycerol monooleate; ursodeoxycholic acid and glycerol distearate; ursodeoxycholic acid and glycerol dioleate; ursodeoxycholic acid and glycerol dilaurate ($\alpha,\alpha'$-dilaurin); ursodeoxycholic acid and polyoxyethylene castor oil; glycyrrhizinic acid and DDPC; glycyrrhizinic acid and DLPC; glycyrrhizinic acid and Tween 20; glycyrrhizinic acid and Tween 40; glycyrrhizinic acid and Tween 60; glycyrrhizinic acid and Tween 80; glycyrrhizinic acid and SPAN 40; glycyrrhizinic acid and SPAN 60; glycyrrhizinic acid and SPAN 80; glycyrrhizinic acid and SPAN 65; glycyrrhizinic acid and SPAN 85; glycyrrhizinic acid and $\alpha$-tocopherol; glycyrrhizinic acid and glycerol monooleate; glycyrrhizinic acid and glycerol distearate; glycyrrhizinic acid and glycerol dioleate; glycyrrhizinic acid and glycerol dilaurate ($\alpha,\alpha'$-dilaurin); and glycyrrhizinic acid and polyoxyethylene castor oil.

More preferred are: cholic acid and DLPC; deoxycholic acid and DDPC; deoxycholic acid and DLPC; deoxycholic acid and Tween 20; deoxycholic acid and Tween 40; deoxycholic acid and Tween 60; deoxycholic acid and Tween 80; deoxycholic acid and SPAN 40; deoxycholic acid and SPAN 60; deoxycholic acid and SPAN 80; deoxycholic acid and SPAN 65; deoxycholic acid and SPAN 80; deoxycholic acid and SPAN 85; deoxycholic acid and $\alpha$-tocopherol; deoxycholic acid and glycerol monooleate; deoxycholic acid and polyoxyethylene castor oil; chenodeoxycholic acid and DLPC; hyodeoxycholic acid and DLPC; glycodeoxycholic acid and DLPC; ursodeoxycholic acid and DDPC; ursodeoxycholic acid and DLPC; ursodeoxycholic acid and Tween 40; ursodeoxycholic acid and Tween 60; ursodeoxycholic acid and Tween 80; ursodeoxycholic acid and SPAN 40; ursodeoxycholic acid and SPAN 65; ursodeoxycholic acid and SPAN 85; ursodeoxycholic acid and $\alpha$-tocopherol; ursodeoxycholic acid and monoolein; ursodeoxycholic acid and polyoxyethylene castor oil; glycyrrhizinic acid and DDPC; glycyrrhizinic acid and DLPC; glycyrrhizinic acid and Tween 40; glycyrrhizinic acid and Tween 60; glycyrrhizinic acid and Tween 80; glycyrrhizinic acid and SPAN 40; glycyrrhizinic acid and SPAN 65; glycyrrhizinic acid and SPAN 85; glycyrrhizinic acid and $\alpha$-tocopherol; glycyrrhizinic acid and glycerol monooleate; and glycyrrhizinic acid and polyoxyethylene castor oil.

Further preferred are: cholic acid and DLPC; deoxycholic acid and DDPC; deoxycholic acid and DLPC; deoxycholic acid and Tween 20; deoxycholic acid and Tween 40; deoxycholic acid and Tween 60; deoxycholic acid and Tween 80; deoxycholic acid and SPAN 40; deoxycholic acid and SPAN 60; deoxycholic acid and SPAN 80; deoxycholic acid and SPAN 65; deoxycholic acid and SPAN 80; deoxycholic acid and SPAN 85; deoxycholic acid and α-tocopherol; deoxycholic acid and glycerol monooleate; deoxycholic acid and polyoxyethylene castor oil; chenodeoxycholic acid and DLPC; hyodeoxycholic acid and DLPC; glycodeoxycholic acid and DLPC; ursodeoxycholic acid and DDPC; ursodeoxycholic acid and DLPC; and glycyrrhizinic acid and DLPC.

[Aluminum-containing substance]

The adjuvant composition of the present invention includes the aforementioned pH-sensitive carrier composed of the amphipathic substance and the pH-sensitive compound, and an aluminum-containing substance as a substance having a stimulus that activates natural immunity (hereinafter referred to a "natural immunity-activating substance").

The aluminum-containing substance is one kind of natural immunity-activating substance. The natural immunity-activating substance means a substance which is recognized by a receptor for structural pattern recognition or which gives a stimulus to immunocompetent cells, leading the immunocompetent cells into an active state.

Herein the term "alum" is used as a generic notation of aluminum-containing adjuvants. Therefore, the "alum" also means an "aluminum-containing substance."

The aluminum-containing substance used in the present invention is not specifically limited so long as it is able to induce cell-mediated immunity when used jointly with the pH-sensitive carrier. However, the aluminum-containing substance is preferably an aluminum salt, examples of which include aluminum hydroxide ($Al(OH)_3$), aluminum phosphate ($AlPO_4$), aluminum sulfate ($Al_2(SO_4)_3$), aluminum potassium sulfate (alum; $AlK(SO_4)_2 \cdot 12H_2O$), and aluminum chloride ($AlCl_3$). Among these, at least one selected from the group consisting of aluminum hydroxide and aluminum phosphate is preferable, from the viewpoint of an effect of inducing cell-mediated immunity. In addition, it is more preferable that the aluminum-containing substance is aluminum hydroxide. Aluminum hydroxide and aluminum phosphate are insoluble in water; when they are used in the adjuvant composition, therefore, it is preferable to use them in the state of being dispersed in a dispersing medium. With such aluminum hydroxide and/or aluminum phosphate used, a gel type adjuvant can be realized.

As the aluminum-containing substance, those aluminum compounds which are commercially available can be used.

Examples of aluminum hydroxide include "Aluminum Hydroxide" made by Sigma-Aldrich, "AlumVax Hydroxide 2%" made by OZ BIOSCIENCES, and "Alhydrogel adjuvant 2%" made by InvivoGen. Among the above-mentioned aluminum hydroxides, "AlumVax Hydroxide 2%" and "Alhydrogel adjuvant 2%" are in the form in which aluminum is dispersed in an aqueous solution in a concentration of approximately 10 mg/mL as metal.

Examples of aluminum phosphate include "Aluminum Phosphate" made by Sigma-Aldrich, "AlumVax Phosphte 2%" made by OZ BIOSCIENCES, and "Adju-Phos" made by Alpha Diagnostic International, Inc. Among the above-mentioned aluminum phosphates, "Adju-Phos" is in the form in which aluminum is dispersed in an aqueous solution in a concentration of approximately 5 mg/mL as metal. Besides, "AlumVax Phosphate 2%" is also in the form in which aluminum phosphate is dispersed in an aqueous solution.

As aluminum sulfate, "Aluminum Sulfate" made by Sigma-Aldrich can be used.

Examples of aluminum potassium sulfate include "alum crystals" made by InvivoGen.

As aluminum chloride, "Aluminum Chloride" made by Sigma-Aldrich can be used.

The "aluminum-containing substance" in the dispersion form can be used as it is in the form of a dispersion. Particularly as for aluminum hydroxide and aluminum phosphate, it is particularly preferable to mix the "aluminum-containing substance" in the form of a dispersion with the pH-sensitive carrier (a dispersion (solution) of the pH-sensitive carrier), from the viewpoint of easy control of coagulated particles of aluminum in the aqueous solution.

In addition, in the case where an aluminum compound in a solid state is used as aluminum hydroxide or aluminum phosphate, it is preferable to disperse the aluminum compound in a dispersing medium in advance before mixing it with the pH-sensitive carrier (a dispersion of the pH-sensitive carrier) and to use it in the form of a dispersion.

In the case of using aluminum sulfate, aluminum potassium sulfate, or aluminum chloride as the aluminum-containing substance, the aluminum-containing compound may be mixed with the pH-sensitive carrier (a dispersion of the pH-sensitive carrier) as it is in a solid state, or the aluminum-containing compound may be mixed with the pH-sensitive carrier (a dispersion of the pH-sensitive carrier) after being dissolved in an aqueous solvent.

The aluminum-containing substances may be used either singly or in combination of two or more of them.

In addition, while the content of the aluminum-containing substance varies depending on the kind of the aluminum-containing substance to be used, the aluminum-containing substance is preferably contained in an amount of 1 to 1,000 μg, more preferably 1 to 500 μg, and further preferably 1 to 100 μg, as aluminum metal, per 100 nmol of the amphipathic substance. When the content of the aluminum-containing substance per 100 nmol of the amphipathic substance is not less than 1 μg, immune response can be suitably induced, which is desirable. On the other hand, when the content of the aluminum-containing substance per 100 nmol of the amphipathic substance is not more than 1,000 μg, side reactions can be reduced, which is desirable. Besides, particularly in the case where the amount of the amphipathic substance contained in 100 μL of the adjuvant composition is less than 10 nmol, the proportion of the aluminum-containing substance per 1 nmol of the amphipathic substance is preferably 10 to 100 μg.

The content (concentration) of the aluminum-containing substance in the adjuvant composition is preferably not less than 1 pg/100 μL, more preferably 1 pg/100 μL to 100 pg/100 μL, and further preferably 10 pg/100 μL to 100 pg/100 μL, as aluminum metal, in the case where the adjuvant composition is in the form of dispersion in an aqueous solvent. When the content of the aluminum-containing substance is not less than 1 pg/100 μL, immune response can be suitably induced, which is desirable. On the other hand, when the content of the aluminum-containing substance is not more than 100 pg/100 μL, side reactions can be reduced, which is desirable.

[Aqueous solvent]

The adjuvant composition may contain an aqueous solvent (hereinafter sometimes referred to as "aqueous medium").

In the case where the adjuvant composition contains an aqueous solvent, the pH-sensitive carrier and the aluminum-containing substance can form a dispersion (hereinafter referred to also as "aqueous solution") in the aqueous solvent.

In this situation, the pH-sensitive carrier preferably forms a complex containing the pH-sensitive compound and the amphipathic substance in the aqueous medium. The form of this complex is not specifically limited, and the pH-sensitive compound and the amphipathic substance may form a membrane. Alternatively, the pH-sensitive compound may be partly or entirely embedded in a structure formed by the amphipathic substance via association and so on. In addition, it is desirable that the pH-sensitive compound and the amphipathic substance form micelle particles (particles in which the pH-sensitive compound and the amphipathic substance are associated into particulate form through hydrophobic interaction, and which are typically particles of a monomolecular film structure). The micelle particles preferably have a diameter of 10 to 200 nm, more preferably 10 to 100 nm, because taking-in by phagocytosis and/or endocytosis is actively performed for particles having a size equal to or larger than a certain size. Note that the aforesaid micelle particles do not include those which form lipid bimolecular membrane structure (such as liposome). Besides, herein the particle diameter of the pH-sensitive carrier is measured by the dynamic light scattering method (with NanoZS90 made by MALVERN Instruments Co., Ltd.).

Further, the adjuvant composition preferably forms a complex (adjuvant complex) which is composed of the pH-sensitive carrier in the form of complex and the aluminum-containing substance in an aqueous medium. Although the form of the complex is not specifically limited, it is preferable that the pH-sensitive compound and the amphipathic substance, which constitute the pH-sensitive carrier, and the aluminum-containing substance form micelle particles. The particle dimeter of the micelle particles is preferably 10 to 200 nm, more preferably 10 to 100 nm.

Note that, in the aqueous solution containing the adjuvant composition, at least one of the pH-sensitive compound, the amphipathic substance, and the substance having an activity to activate natural immunity may remain in a free state without forming an associated body.

The solvent of the aqueous solution containing the adjuvant composition of the present invention is preferably an aqueous solution containing a buffering agent, NaCl, and a sugar such as glucose and sucrose.

The buffering agent is not specifically limited; any known one may be used so long as it can keep the pH of the adjuvant composition equal to or higher than the physiological pH. Examples of the usable buffering agent include phosphate buffer, citrate buffer, citrate-phosphate buffer, tris(hydroxymethyl)aminomethane-HCl buffer (tris-hydrochloric acid buffer), MES buffer (2-morpholinoethane sulfonate buffer), TES buffer (N-tris(hydroxymethyl)methyl-2-aminoethane sulfonate buffer), acetate buffer, MOPS buffer (3-morpholinopropane sulfonate buffer), MOPS-NaOH buffer, HEPES buffer (4-(2-hydroxyethyl)-1-peperazine ethanesulfonate buffer), HEPES-NaOH buffer and other GOOD buffers, amino acid buffer such as glycine-hydrochloric acid buffer, glycine-NaOH buffer, glycylglycine-NaOH buffer, and glycylglycine-KOH buffer; boric acid-based buffer such as Tris-borate buffer, borate-NaOH buffer, and borate buffer; and imidazole buffer. Preferable among these buffers are phosphate buffer, citrate buffer, citrate-phosphate buffer, Tris-hydrochloric acid buffer, MES buffer, acetate buffer, and HEPES-NaOH buffer. These buffers may be used in any concentration without specific restrictions. An adequate concentration is 0.1 to 200 mM, preferably 1 to 100 mM. Note that the term "concentration of buffer" used herein refers to the concentration (mM) of the buffer contained in the aqueous solution.

The concentrations of NaCl and a sugar such as glucose and sucrose are not particularly limited, and they are preferably 0.1 to 200 mM, more preferably 1 to 150 mM.

The concentration of the pH-sensitive carrier in the aqueous solution is not specifically limited. However, it is preferable that the total molar concentration of the pH-sensitive compound and the amphipathic substance is not less than 7.3 μmol/L, more preferably not less than 8.0 μmol/L, further preferably not less than 50 μmol/L, particularly

preferably not less than 100 μmol/L, and most preferably not less than 1 mmol/L. Besides, it is preferable that the total molar concentration of the pH-sensitive compound and the amphipathic substance is not more than 500 mmol/L, more preferably not more than 300 mmol/L, further preferably not more than 280 mmol/L, particularly preferably not more than 6.5 mmol/L, and most preferably not more than 4.2 mmol/L.

Furthermore, in a particularly preferable embodiment from the viewpoint of reducing side reactions, the total molar concentration of the pH-sensitive compound and the amphipathic substance is preferably 100 μmol/L to 300 mmol/L, more preferably 1 to 280 mmol/L.

In addition, the aluminum-containing substance in the aqueous solution is preferably used in an amount of 1 pg/100 μL to 100 pg/100 μL, more preferably 10 pg/100 μL to 100 pg/100 μL, as aluminum metal.

[Other components]

The adjuvant composition may further contain other components. While the other components are not specifically limited, examples of them include a stabilizer.

The stabilizer is not particularly limited so long as it does not adversely affect the pH-sensitive carrier or the aluminum-containing substance, and known stabilizers can be used, examples of which include saturated or unsaturated alcohols having 4 to 20 carbon atoms, such as 1-octanol, 1-dodecanol, 1-hexadodecanol, and 1-eicosanol; saturated or unsaturated fatty acids having 12 to 18 carbon atoms, such as lauric acid, myristic acid, palmitic acid, stearic acid, and oleic acid; alkyl esters (alkyl having 1 to 3 carbon atoms) of saturated or unsaturated fatty acids having 8 to 18 carbon atoms, such as methyl caprylate (methyl octanoate), ethyl caprylate (ethyl octanoate), methyl laurate, ethyl laurate, ethyl myristate, ethyl palmitate, ethyl stearate, methyl oleate, and ethyl oleate; D(L)-amino acids, such as D(L)-alanine, arginine, asparagine, aspartic acid, cysteine, glutamine, glycine, histidine, leucine, isoleucine, lysine, methionine, proline, serine, threonine, tryptophan, tyrosine, valine, and phenylalanine; amino acid triglycerides, such as tricaproin and tricaprylin; polyoxyethylene sorbitan trifatty acid esters having 12 to 18 carbon atoms, such as polyoxyethylene sorbitan tripalmitic acid ester and polyoxyethylene sorbitan trioleic acid ester (for example, Tween 65 and Tween 85); polyoxyethylene alkyl esters having 12 to 18 carbon atoms, such as polyoxyethylene lauric acid ester, polyoxyethylene myristic acid ester, polyoxyethylene palmitic acid ester, and polyoxyethylene stearic acid ester (for example, PEG20 stearyl ether and PEG23 lauryl ether); polyoxyalkylene hardened castor oil (for example, PEG10 hardened castor oil, PEG40 hardened castor oil, and PEG60 hardened castor oil); saturated or unsaturated monofatty acid glycerol ester having 8 to 18 carbon atoms, such as caprylin (glycerol octanoate), glycerol monocaprate, glycerol monolaurate, glycerol monomyristate, glycerol monopalmitate, glycerol monostearate, and glycerol monooleate; difatty acid glycerol having 8 to 16 carbon atoms, such as glycerol dioctanoate, glycerol dicaprate, glycerol dilaurate, glycerol dimyristate ester, and glycerol dipalmitate; α-tocopherol acetic acid ester, castor oil, soybean oil, cholesterol, squalene, squalane, lactose, ascorbyl palmitate, benzyl benzoate, methyl paraoxybenzoate, ethyl paraoxybenzoate, propyl paraoxybenzoate, and butyl paraoxybenzoate. Note that the "carbon number" in the stabilizer means the number of carbon atoms in the fatty acid component (acyl group) constituting the hydrophobic moiety.

The content of these other components is not particularly limited so long as it does not adversely affect the pH-sensitive carrier or the aluminum-containing substance, but the content is preferably not more than 150 mol, more preferably more than 0 mol and not more than 66.4 mol, per 100 mol of the amphipathic substance.

The adjuvant composition according to the present aspect can effectively induce CTL by being administered together with an antigen.

Specifically, in the adjuvant composition according to the present aspect, even when the aluminum-containing substance is used jointly with the pH-sensitive carrier, the function of the pH-sensitive carrier, for example, the effect of promoting the membrane-disruption function (and the effect of promoting the membrane-fusion function) can be exhibited suitably. In addition, when the aluminum-containing substance is used together with the pH-sensitive carrier, the function of the aluminum-containing substance can also be exhibited suitably. The reason for this has not yet been elucidated, but the reason is inferred to be as follows.

The pH-sensitive carrier in its desirable form includes the pH-sensitive compound and the amphipathic substance, and has the effect of promoting the membrane-disruption function (in some cases, both the effect of promoting the membrane-disruption function and the effect of promoting the membrane-fusion function). In this instance, as aforementioned, the effect of promoting the membrane-disruption function (and the effect of promoting the membrane-fusion function) is based on a change in the association state of the pH-sensitive carrier, caused by the pH-sensitive compound in an acidic environment, and the rearrangement with the cell membrane, such as endosome, brought about by the amphipathic substance in this situation. Here, the use of the aluminum-containing substance jointly with the pH-sensitive carrier does not change the pH sensitivity of the pH-sensitive compound, so that the pH-sensitive compound can bring about a change in the association state of the pH-sensitive carrier. In addition, whether the aluminum-containing substance may, for example, be incorporated in the amphipathic substance or be present independently from the pH-sensitive carrier, the rearrangement with the cell membrane by the amphipathic substance is not affected. Then, even

15

with the aluminum-containing substance used jointly with the pH-sensitive carrier, the function of the pH-sensitive carrier is not impaired. Besides, the aluminum-containing substance is, for example, only incorporated in the amphipathic substance of the pH-sensitive carrier by the hydrophobic interaction, for example, or only present independently from the pH-sensitive carrier, so that its function is also not impaired. As a result, the adjuvant composition according to the present aspect, when administered together with an antigen, can introduce the antigen into a cytoplasmic substrate by the function of the pH-sensitive carrier, and, at the same time, can suitably induce cross-presentation based on the antigen introduced into the cytoplasmic substrate, by the action of the aluminum-containing substance, whereby cell-mediated immunity (CTL) can be induced effectively.

Note that the aforesaid reason is merely an inferred one, and any other effects produced by other causes will be included in the technical scope of the present invention.

According to a preferred embodiment of the present invention, humoral immunity can also be induced suitably.

As aforementioned, an exogenous antigen is usually decomposed into peptide fragments by endosomes in antigen presenting cells and forms a complex with MHC class II molecules, to be presented to the CD4 positive T cells.

In the case where the adjuvant composition according to the present aspect induces CTL through cross-presentation of the antigen administered together with it, the antigen and the aluminum-containing substance may be introduced into a cytoplasmic substrate when rearrangement of the cell membrane of endosomes occurs. In one embodiment, however, even if the rearrangement occurs, the antigen and the aluminum-containing substance may partly or entirely remain in the endosomes. Besides, in one embodiment, in the case where the antigen and the adjuvant composition exist independently, taking-in of only the antigen may occur in part of the endosomes. Then, the antigen is decomposed into peptide fragments in the endosomes and forms a complex with MHC class II molecules, to be presented to CD4 positive T cells, whereby humoral immunity is induced. In this instance, since the dendritic cells inducing the cross-presentation suitably are in an immunologically activated state, the induction of the humoral immunity is brought about suitably. Alternatively, the dendritic cells inducing the cross-presentation suitably produce cytokine (for example, IFNγ) actively that activates immunity, thereby making the environment suitable for induction of immunity.

Therefore, according to another embodiment of the present invention, humoral immunity can be induced, together with cross-presentation or in place of cross-presentation.

<Vaccine composition>

The vaccine composition includes an adjuvant composition and an antigen.

[Adjuvant composition]

As the adjuvant composition, the same one as aforementioned can be used, and, hence, description thereof is omitted here.

[Antigen]

The antigen is not specifically limited so long as it produces immune response, and it is preferably a peptide or a protein.

Examples of the peptide or protein include viral antigen, bacterial antigen, mycotic antigen, protozoan or verminous antigen, cancer antigen, allergy-related antigen, disease-related antigen, and graft rejection-related antigen.

The viral antigen is not particularly limited, and examples thereof include: human immunodeficiency virus (HIV) antigen, such as gene product of gag, pol, and env genes, Nef protein, reverse transcriptase, and other HIV components; hepatitis virus antigen, such as S, M, and L proteins of hepatitis B virus, pre-S antigen of hepatitis B virus, hepatitis C virus RNA, and virus components of hepatitis A, B, and C; influenza virus antigen, such as hemagglutinin, neuraminidase, and other influenza virus components; measles virus antigen; rubella virus antigen; rotavirus antigen; cytomegalovirus antigen; respiratory syncytial virus antigen; herpes simplex virus antigen; varicella zoster virus antigen; Japanese encephalitis virus antigen; and rabies virus antigen. Other examples include peptides derived from adenovirus, retrovirus, picornavirus, herpesvirus, rotavirus, hantavirus, coronavirus, togavirus, flavivirus, rhabdovirus, paramyxovirus, orthomyxovirus, bunyavirus, arenavirus, reovirus, papillomavirus, parvovirus, poxvirus, hepadnavirus, or spongy virus.

The bacterial antigen is not specifically limited, and examples thereof include bacterial antigen, such as pertussis toxin, filamentous hemagglutinin, pertactin, FIM2, FIM3, cyclase adenylate, and other pertussis bacterial antigen components; diphtheria bacterial antigen, such as diphtheria toxin or toxoid, and other diphtheria bacterial antigen components; bacterial antigen of tetanus bacillus and streptococcus, such as tetanus toxin or toxoid, and other tetanus bacterial antigen components; Gram-negative bacillary bacterial antigen, such as lipopolysaccharide, and other Gram-negative bacterial antigen components; tubercle bacillus bacterial antigen, such as mycolic acid, heat-shock protein 65 (HSP 65), 30 kDa major secretory protein, antigen 85A, and other mycobacteria antigen components; helicobacter and pylori bacterial antigen components; pneumococcus bacterial antigen; influenza bacillus bacterial antigen; anthrax bacillus bacterial antigen; and rickettsia bacterial antigen.

The mycotic antigen is not particularly limited, and examples thereof include candida mycotic antigen components; histoplasma mycotic antigen; Cryptococcus mycotic antigen; coccidioides mycotic antigen; and ringwork mycotic antigen.

The protozoan or verminous antigen is not specifically limited, and examples thereof include plasmodium falciparum antigen; toxoplasma antigen; schistosoma antigen; Leishmania antigen; and trypanosome cruzi antigen.

The cancer antigen is not particularly limited, and examples thereof include those cancer antigens derived from the cell surface of cells of tumor tissue, protoplasm, nuclei, and cell organelles. Examples of the cancer include leukemia, lymphoma, nervous tumor, melanoma, breast cancer, lung cancer, head and neck cancer, stomach cancer, colon cancer, liver cancer, pancreatic cancer, cancer of the uterine cervix, uterine cancer, ovarian cancer, vaginal cancer, testis cancer, prostatic cancer, penile cancer, bone tumors, hemangioma, lip cancer, cancer of epipharynx, pharyngeal cancer, carcinoma of esophagus, rectal cancer, carcinoma of the gallbladder, cancer of the bile duct, laryngeal cancer, lung cancer, bladder carcinoma, kidney cancer, brain tumor, thyroid carcinoma, Hodgkin's disease, and non-Hodgkin lymphoma. Note that typical examples of the cancer antigen include HER2/neu (Human EGFR related 2), CEA (Carcinogenic Embryonic Antigen), MAGE (Melanoma-associated Antigen), XAGE (X antigen family member), NY-ESO-1, gp100, Melan/mart-1, Tyrosinase, PSA (Prostate Specific Antigen), PAP (Prostate Acid Phosphatase), p53, K-ras, N-ras, Bcr-Abl, MUC-1 (Mucin-1), PSMA (Prostate Specific Membrane Antigen), surviving, WT-1 (Wilms tumor suppressor gene 1), AFP (Alpha Fetoprotein), GPC (Glypican), and EGFR (Epidermal Growth Factor Receptor).

The allergy-related antigen is not particularly limited, and examples thereof include pollen antigen, such as cedar pollen antigen, ragweed pollen antigen, and ryegrass pollen antigen; animal-derived antigen, such as rat mite antigen and cat antigen; histocompatible antigen; and therapeutic drug such as penicillin.

The disease-related antigen (autoimmune disease, allergy, etc.) is not specifically limited, and examples thereof include diabetes, articular rheumatism, myasthenia gravis, systemic lupus erythematosus, atopic dermatitis, psoriasis, Sjogren's syndrome, alopecia areata, Crohn's disease, ulcerative colitis, conjunctivitis, keratoconjunctivitis, asthma, allergic asthma, proctitis, drug eruption, allergic encephalomyelitis, acute necrotizing hemorrhagic encephalopathy, hypoplastic anemia, erythroblast anemia, idiopathic thrombocytopenia, Wegener granulomatosis, Stevens-Johnson syndrome, and interstitial pulmonary fibrosis. Specific examples of the antigen related to autoimmune disease include glutamic acid decarboxylase 65 (GA9D 65), natural DNA, myelin basic protein, myelin proteolipid protein, acetylcholine receptor components, thyroglobulin, and thyroid stimulating hormone (TSH) receptor.

The graft rejection-related antigen is not particularly limited, and examples thereof include the antigenic components of the graft to be transferred to the graft recipient, such as graft components for the heart, lung, liver, pancreas, kidney, and nerve.

The aforementioned antigens may be used either singly or in combination of two or more of them.

The content of the antigen is preferably 3.2 to 400 $\mu$g, more preferably 16 to 80 $\mu$g, per 100 nmol of the amphipathic substance constituting the pH-sensitive carrier.

The rate in which the antigen is to be incorporated is not particularly limited; while the antigen and the adjuvant composition may exist independently, the incorporation rate is preferably not less than 3%, more preferably 5% to 80%, and further preferably 10% to 60%. When the incorporation rate is not less than 3%, for example, when endocytosis of the vaccine composition into cells occurs, the antigen is highly possibly introduced into the endosome into which the adjuvant composition is introduced, and the effect of the invention can be suitably obtained, which is desirable. Note that the "incorporation rate of the antigen" means mainly the proportion in which the antigen is supported on or embraced in the adjuvant composition, and, as the value of the incorporation rate, the values measured by the method described in Examples are to be adopted.

[Additives]

The vaccine composition may contain other pharmaceutical additives.

The usable additives vary depending on the dosage form of the vaccine composition. In this instance, the vaccine composition may be a solid preparation, such as tablet, powder, and capsule or may be a liquid preparation, such as an injection preparation, but is preferably a liquid preparation. Note that, in the case of a liquid preparation, it may be supplied as a dried product which is regenerated with water or other suitable excipient at the time of use.

In the case where the vaccine composition is in the form of tablets or capsules, it is desirably provided with enteric coating by an ordinary method. As the enteric coating, those which are in ordinary use in the field concerned can be utilized. Besides, the capsules can contain either of powder and liquid.

Besides, in the case where the vaccine composition is a solid preparation, it may contain excipient (for example, sugar such as lactose, sucrose, etc., starch such as corn starch, etc., celluloses such as crystalline cellulose, etc., gum arabic, magnesium metasilicate aluminate, calcium phosphate, and the like); lubricant (for example, magnesium stearate, talc, polyethylene glycol, and the like); binder (for example, sugar such as mannitol, sucrose, etc., crystalline cellulose, polyvinylpyrrolidone, hydroxypropylmethyl cellulose, and the like); disintegrator (for example, starches such as potato starch, etc., celluloses such as carboxymethyl cellulose, etc., crosslinked polyvinylpyrrolidone, etc.); coloring agent, and corrigent.

On the other hand, in the case where the vaccine composition is a liquid preparation, it may contain solvent (for example, physiological saline solution, sterilized water, buffer solution, etc.), membrane stabilizer (for example, cholesterol, etc.), tonicity adjusting agent (for example, sodium chloride, glucose, glycerin, etc.), antioxidant (for example, tocopherol, ascorbic acid, glutathione, etc.), and antiseptics (for example, chlorobutanol, paraben, etc.). Noe that the solvent may be the solvent that is used for preparation of the vaccine composition.

According to one embodiment of the present invention, the vaccine composition can induce cell-mediated immunity through effective cross-presentation of the antigen. This permits the induction of CTL, for example. The term "to induce cell-mediated immunity" herein means that formation of much spots can be obtained in the ELIspot method described herein, as compared to a control, which is not treated with the vaccine composition (namely, which is a mixture of the antigen and the aluminum-containing substance).

In addition, according to another embodiment of the present invention, the vaccine composition can induce humoral immunity. This enables production of an antibody such as IgG. In this instance, the term "to induce humoral immunity" means that a high IgG antibody titer is obtained as compared to a control, to which the antigen is administered.

[Use]

The vaccine composition of the present aspect, when administered to a subject and with the environment thereof decreasing in pH below the physiological pH (for example, pH 6.5), shows the effect of promoting the membrane-disruption function, or the effect of promoting the membrane-disruption function and the effect of promoting the membrane-fusion function, thereby permitting the antigen to be effectively released to the cytoplasmic substrate. Then, cell-mediated immunity can be induced suitably, and immunity can be imparted.

According to one aspect of the present invention, therefore, there is provided a method for treating or preventing a disease, the method including administering an effective amount of the vaccine composition to a subject who needs the treatment or prevention of a disease.

The method for administering the vaccine composition is not specifically limited, and examples thereof include oral administration; parenteral administration such as intravenous injection, arterial injection, subcutaneous injection, intracutaneous injection, intramuscular injection, intrathecal injection, dermal administration or percutaneous absorption, etc. For example, in the case of using a peptide or a protein as the antigen, preferred is administration through a parenteral route, particularly, administration by subcutaneous injection, intracutaneous injection, intramuscular injection, or intravenous injection. Note that a vaccine composition in which the antigen is independently mixed without being supported on or embraced in the adjuvant composition is preferably administered in the form of local administration, specifically, subcutaneous administration, intracutaneous administration, or intramuscular administration.

The above-mentioned subject is preferably a mammal, especially preferably a human.

Examples of the aforesaid diseases include: virus infectious disease such as human immunodeficiency syndrome (HIV), hepatitis, and influenza; bacterial injection such as pertussis, diphtheria, tetanus, tuberculosis, Helicobacter pylori, and microbism caused by pneumococcus; mycosis such as candida; protozoiassis or vermiculous infection such as malaria; cancer such as leukemia, lymphoma, nervous tumor, melanoma, breast cancer, lung cancer, head and neck cancer, stomach cancer, colon cancer, liver cancer, pancreatic cancer, cancer of the uterine cervix, uterine cancer, ovarian cancer, vaginal cancer, testis cancer, prostatic cancer, penile cancer, osteoncus, hemangioma, lip cancer, cancer of epipharynx, pharyngeal cancer, carcinoma of the esophagus, rectal cancer, carcinoma of the gallbladder, cancer of the bile duct, laryngeal cancer, lung cancer, bladder carcinoma, kidney cancer, brain tumor, thyroid carcinoma, Hodgkin's disease, and non-Hodgkin lymphoma; allergy caused by cedar pollen; autoimmune disease such as diabetes, articular rheumatism, myasthenia gravis, and systemic lupus erythematosus; and graft rejection such as graft-versus-host disease (GVHD).

According to one preferable embodiment of the present invention, there is provided a method for treating or preventing a disease. For such a finding, the common technical knowledge at the time of application can be referred to, as required.

In addition, in one embodiment of the present invention, the vaccine composition permits the antigen to be transported to cells by cultivation. In other words, according to one aspect of the present invention, there is provided a method for cultivation for transporting the antigen to cells.

The method for cultivation includes a step of cultivating cells in a culture medium containing the vaccine composition.

The culture medium is not specifically limited, and known ones can be used. Specific examples of the usable culture medium include MEM, DMEM, and RPMI.

The amount of the vaccine composition to be added to the culture medium is not particularly limited, but the molar concentration of the amphipathic substance is preferably 0.66 µmol/L to 1.0 mmol/L, more preferably 6.6 µmol/L to 0.88 mmol/L, and further preferably 7.2 µmol/L to 0.56 mmol/L.

In addition, the pH of the culture medium is preferably not less than 7.0, and more preferably 7.2 to 7.8. When the pH of the culture medium is not less than 7.0, the pH-sensitive compound constituting the pH-sensitive carrier can be prevented from becoming unstable in the culture medium, which is preferable.

The cells are not specifically limited, and examples thereof include those cells collected from the subject, and established cultured cells.

In this instance, specific examples of the cells collected from the subject or the established cultured cells include dendritic cells, NK (Natural Killer) cells, T cells, B lymphocytes, and lymphoblasts.

Among the aforesaid cells, preferable for use are the cells collected from the subject, more preferable for use are the dendritic cells, NK cells, T cells, lymphoblasts and the like collected from the subject, and further preferable for use are dendritic cells.

In the case of using the cells collected from the subject, the cells are collected from the subject by blood collection, biopsy or the like. In other words, in one embodiment, the method for cultivation may include a step of collecting cells from the subject.

Note that the cultured cells may be administered to the subject. This enables therapy or prevention of a disease in the subject. Thus, in one embodiment of the present invention, there is provided a method for treating or preventing a disease.

In one preferable embodiment, the method for therapy or prevention includes a step of collecting cells from the subject, a step of cultivating the collected cells in a culture medium containing the vaccine composition, and a step of administering the cultured cells to the subject.

This enables therapy or prevention of a disease. Note that the disease is as aforementioned.

<Method of producing adjuvant composition>

The adjuvant composition according to the present invention can be produced by various methods without specific restrictions.

For instance, the adjuvant composition in which the pH-sensitive carrier and the aluminum-containing substance exist independently can be produced by mixing the pH-sensitive carrier and the aluminum-containing substance. In addition, the adjuvant composition in which the aluminum-containing substance is supported on or embraced in the pH-sensitive carrier can be produced by associating the pH-sensitive carrier and the aluminum-containing substance with each other.

The following is a detailed description of a preferred embodiment of using a pH-sensitive carrier which includes a predetermined pH-sensitive compound and a predetermined amphipathic substance and which has the effect of promoting the membrane-disruption function, as the pH-sensitive carrier.

The method of producing the adjuvant composition according to the present aspect includes a step of associating at least one pH-sensitive compound selected from the group consisting of deoxycholic acid, cholic acid, ursodeoxycholic acid, chenodeoxycholic acid, hyodeoxycholic acid, glycodeoxycholic acid, glycyrrhizinic acid, glycyrrhetinic acid, and salts thereof, at least one amphipathic substance selected from the group consisting of phosphatidylcholine having only an acyl group having 10 to 12 carbon atoms, polyoxyethylene sorbitan monofatty acid ester having an acyl group having 12 to 18 carbon atoms, sorbitan fatty acid ester having only an acyl group having 16 to 18 carbon atoms, glycerol monooleate, glycerol dilaurate, glycerol distearate, glycerol dioleate, polyoxyethylene castor oil, and α-tocopherol, and an aluminum-containing substance with one another.

The method for associating the pH-sensitive compound, the amphipathic substance and the aluminum-containing substance with one another may sufficiently be accomplished by contact of the pH-sensitive compound, the amphipathic substance, and the aluminum-containing substance with one another in an aqueous solution. Therefore, the adjuvant composition according to the present aspect can be produced by bringing the pH-sensitive compound, the amphipathic substance, and the aluminum-containing substance into contact with one another in an aqueous solution.

The method for bringing the pH-sensitive compound, the amphipathic substance, and the aluminum-containing substance into contact with one another in an aqueous solution is not specifically limited so long as they form an associated body. Examples of the method include (1) <a> a method in which an aqueous solution containing the pH-sensitive compound, an aqueous solution containing the amphipathic substance, and an aqueous solution containing the aluminum-containing substance are separately prepared, these aqueous solutions are mixed together, and the resulting solution is vigorously stirred for dispersion by use of an emulsifier or the like to obtain the adjuvant composition; <b> a method in which an aqueous solution containing the pH-sensitive compound and an aqueous solution containing the amphipathic substance are mixed together, the resulting solution is vigorously stirred for dispersion by use of an emulsifier or the like to obtain a dispersion of the pH-sensitive carrier, thereafter the dispersion is mixed with the aluminum-containing substance or an aqueous solution containing the aluminum-containing substance, to obtain the adjuvant composition; <c> a method in which the pH-sensitive compound and the amphipathic substance are dispersed in an aqueous solution, the solution is vigorously stirred for dispersion by use of an emulsifier or the like to obtain a dispersion of the pH-sensitive carrier, thereafter the dispersion is mixed with the aluminum-containing substance or an aqueous solution containing the aluminum-containing substance, to obtain the adjuvant composition; and (2) a method of preparing the adjuvant composition by the Bangham method, which is known as a method for producing liposome. Note that, as a specific method of the Bangham method, it can be carried out, for example, in the following way. That is, first, the constituents of the pH-sensitive carrier are dissolved in an organic solvent (e.g., methanol or chloroform) in a glass container, and the organic solvent is removed by a rotary evaporator or the like, causing a thin film to be formed on the wall of the glass container. Next, an aqueous solution is added to the glass container in which the thin film has been formed, the thin film is thereby swelled at 5°C to 35°C, and thereafter the glass container is shaken at 5°C to 35°C. In this instance, the thin film can be sufficiently dispersed in the aqueous solution by vigorous stirring with an emulsifier, vortex mixer, ultrasonic waves or the like. With the thin film component dispersed in the aqueous solution, an aqueous solution containing the pH-sensitive carrier (a dispersion of the pH-sensitive carrier) is obtained. The aqueous solution containing the pH-sensitive carrier (the dispersion of the pH-sensitive carrier) and an aqueous solution containing the aluminum-containing substance are mixed with each other, whereby the adjuvant composition is obtained. Here, in the case where the aqueous solution containing the aluminum-containing substance is used as the aqueous solution to be added to the thin film, the adjuvant composition can be obtained by dispersing the thin film. The details of the Bangham method can be found by referring to known methods for production of liposome, and are described in "Liposome" (complied by Shoshichi Nojima, Junzo Sunamoto, and Keizo Inoue, published by Nankoudo), and "Liposome in Life Science" (compiled by Hiroshi Terada and Tetsuro Yoshimura, published by Springer-Verlap, Tokyo).

In the aforesaid production method (1), the vigorous dispersing in preparing the dispersion may be accomplished by use of an emulsifier, a vortex mixer, ultrasonic waves or the like. Besides, the aqueous solution containing the amphipathic substance may be mixed with the pH-sensitive compound and the aluminum-containing substance. Note that, as the solvent for the aqueous solution, the solvents for the aqueous solution mentioned above can be used. In the aforesaid method (1), the temperatures in preparing the aqueous solutions and the temperature in mixing the aqueous solutions are not particularly limited, and are preferably 5°C to 35°C, more preferably a normal temperature of 15°C to 25°C.

Note that the method for adding other components such as stabilizer, which may be contained in the adjuvant composition containing the aqueous solvent as a constituent, is not specifically limited. For instance, the other components may be added to the aqueous solution containing the pH-sensitive compound, the aqueous solution containing the amphipathic substance, and/or the aqueous solution containing the aluminum-containing substance. Alternatively, at the time of preparing the thin film by the Bangham method, the other components may be dissolved together with the constituents of the adjuvant composition, and the thin film containing these components may be used to thereby obtain the aqueous solution containing the adjuvant composition.

As the aqueous solution containing the pH-sensitive compound, the aqueous solution containing the amphipathic substance, and the aqueous solution containing the aluminum-containing substance, there can be used solutions similarly those used in the "method for producing the vaccine composition" to be described later or those prepared by reference to the producing method.

<Method for producing vaccine composition>

The method for producing the vaccine composition according to the present invention is not specifically limited, and the vaccine composition can be produced by various methods. The method for producing the vaccine composition according to one embodiment includes: a step of associating at least one pH-sensitive compound selected from the group consisting of deoxycholic acid, cholic acid, ursodeoxycholic acid, chenodeoxycholic acid, hyodeoxycholic acid, glycodeoxycholic acid, glycyrrhizinic acid, glycyrrhetinic acid, and salts thereof, at least one amphipathic substance selected from the group consisting of phosphatidylcholine having only an acyl group having 10 to 12 carbon atoms, polyoxyethylene sorbitan monofatty acid ester having an acyl group having 12 to 18 carbon atoms, sorbitan fatty acid ester having only an acyl group having 16 to 18 carbon atoms, glycerol monooleate, glycerol dilaurate, glycerol distearate, glycerol dioleate, polyoxyethylene castor oil, and $\alpha$-tocopherol, and an aluminum-containing substance with one another; and a step of mixing an antigen with an associated body obtained by the association.

In the step of mixing the antigen with the associated body, it is sufficient to mix the antigen into an aqueous solution containing the associated body. For instance, the adjuvant composition obtained by the aforesaid method (1) or (2) and the antigen may be mixed with each other, whereby the vaccine composition can be obtained. Examples of the usable method include: (V-1) <a> a method in which an aqueous solution containing the pH-sensitive compound, an aqueous solution containing the amphipathic substance, an aqueous solution containing the aluminum-containing substance, and an aqueous solution containing the antigen are separately prepared, these aqueous solutions are mixed, and the resulting solution is vigorously stirred for dispersion by use of an emulsifier or the like, to obtain the vaccine composition; <b> a method in which an aqueous solution containing the pH-sensitive compound and an aqueous solution containing the amphipathic substance are mixed, the resulting solution is vigorously stirred for dispersion by use of an emulsifier or the like to obtain a dispersion of the pH-sensitive carrier, thereafter the dispersion is mixed with the aluminum-containing substance (or an aqueous solution containing the same) and the antigen (or an aqueous solution containing the same), to obtain the vaccine composition; <c> a method in which the pH-sensitive compound and the amphipathic substance are dispersed in an aqueous solution, the resulting solution is vigorously stirred for dispersion by use of an emulsifier or the like to obtain a dispersion of the pH-sensitive carrier, and thereafter the dispersion is mixed with the aluminum-containing substance (or an aqueous solution containing the same) and the antigen (or an aqueous solution containing the same), to obtain the vaccine composition; and (V-2) a method in which an adjuvant composition obtained by the aforesaid Bangham method of (2) is mixed with the antigen or an aqueous solution containing the antigen, to obtain the vaccine composition. In this instance, a solution containing the aluminum-containing substance and the antigen may be used as the aqueous solution to be added to the thin film formed from the constituents of the pH-sensitive carrier. Besides, an aqueous solution containing the antigen may be used as the aqueous solution to be added to the thin film, and, after that, an aqueous solution containing the aluminum-containing substance may be mixed therewith.

The temperature at which the aqueous solution containing the antigen is mixed with the adjuvant composition or the dispersion of the pH-sensitive carrier is not particularly limited, and is preferably 5°C to 35°C, more preferably a normal temperature of 15°C to 25°C.

As the aqueous solution containing the pH-sensitive compound, the aqueous solution containing the amphipathic substance, the aqueous solution containing the aluminum-containing substance, the aqueous solution containing the antigen, and the aqueous solution containing the antigen and the aluminum-containing substance, there can be used those prepared as follows.

(Aqueous solution containing pH-sensitive compound)

The solution containing the pH-sensitive compound contains the pH-sensitive compound and a solvent. Besides, it may contain additives, as required.

As the pH-sensitive compound, those which have been mentioned above can be used, and, hence, description thereof is omitted here.

As the solvent, there can be used sterilized water and the like, as well as aqueous solutions containing a buffering agent, NaCl and sugar such as glucose and sucrose. Among these, preferred for use are physiological saline solution, sterilized water, and buffers, from the viewpoint of suitable administration of the vaccine composition to a living body.

In the aqueous solution containing the pH-sensitive compound, the molar concentration of the pH-sensitive compound is preferably not less than 5 $\mu$mol/L, more preferably not less than 10 $\mu$mol/L, further preferably not less than 50 $\mu$mol/L, particularly preferably not less than 100 $\mu$mol/L, and most preferably not less than 1 mmol/L. Besides, the molar concentration of the pH-sensitive compound in the aqueous solution is preferably not more than 500 mmol/L, more preferably not more than 300 mmol/L, further preferably not more than 250 mmol/L, and particularly preferably not more than 200 mmol/L.

Further, from the viewpoint of reducing side reactions, the molar concentration of the pH-sensitive compound is preferably 100 $\mu$mol/L to 300 mmol/L, more preferably 1 to 200 mmol/L.

(Aqueous solution containing amphipathic substance)

The solution containing the amphipathic substance contains the amphipathic substance and a solvent. Besides, it may contain additives, as required.

As the amphipathic substance and the solvent, those which have been mentioned above can be used, and, hence, description thereof is omitted here.

In the aqueous solution containing the amphipathic substance, the molar concentration of the amphipathic substance is preferably not less than 5 $\mu$mol/L, more preferably not less than 10 $\mu$mol/L, further preferably not less than 50 $\mu$mol/L, particularly preferably not less than 100 $\mu$mol/L, and most preferably not less than 500 $\mu$mol/L. Besides, the molar concentration of the amphipathic substance in the aqueous solution is preferably not more than 500 mmol/L, more preferably not more than 300 mmol/L, further preferably not more than 250 mmol/L, particularly preferably not more than 200 mmol/L, and most preferably not more than 150 mmol/L.

Further, from the viewpoint of reducing side reactions, the molar concentration of the amphipathic substance is preferably 50 $\mu$mol/L to 200 mmol/L, more preferably 500 $\mu$mol/L to 150 mmol/L.

(Aqueous solution containing aluminum-containing substance)

The solution containing the aluminum-containing substance contains the aluminum-containing substance and a solvent. Besides, it may contain additives, as required.

As the aluminum-containing substance and the solvent, those which have been mentioned above can be used, and, hence, description thereof is omitted here.

The concentration of aluminum in the aqueous solution containing the aluminum-containing substance is preferably 1 pg/100 μL to 100 pg/100 μL, more preferably 10 pg/100 μL to 100 pg/100 μL, as aluminum metal.

(Aqueous solution containing antigen)

The solution containing the antigen contains the antigen and a solvent.

As the antigen and the solvent, those which have been mentioned above can be used, and, hence, description thereof is omitted here.

Mixing

The method for mixing the aqueous solution containing the pH-sensitive compound, the aqueous solution containing the amphipathic substance, the aqueous solution containing the aluminum-containing substance, and the aqueous solution containing the antigen which is aforementioned is not specifically limited.

It is preferable to disperse the constituents by stirring the mixed liquid obtained, and the stirring can be carried out by use of, for example, an emulsifier, a vortex mixer, ultrasonic waves, or the like.

Note that in one embodiment, the aqueous solution containing the pH-sensitive compound, the aqueous solution containing the amphipathic substance, the aqueous solution containing the aluminum-containing substance, and the aqueous solution containing the antigen may be in the form of a mixed liquid obtained by combining two or more of the constituents, without being made to be separate solutions. For example the aqueous solution containing the pH-sensitive compound and the aqueous solution containing the aluminum-containing substance are prepared, and then this prepared solution may be mixed with the aqueous solution containing the amphipathic substance and the aqueous solution containing the antigen.

EXAMPLES

The present invention will be described in more detail below by way of Examples, but the present invention is not to be limited by these Examples in any way.

<Raw materials>

In the Examples, the following compounds were used. In the case where the reagent name is identical with the product name, the product name is omitted.

(1) pH-sensitive compound, amphipathic substance, etc.

·Sodium deoxycholate (from Nacalai Tesque Inc.)
·Sodium cholate (from Nacalai Tesque Inc.)
·Sodium ursodeoxycholate (from Tokyo Chemical Industry Co., Ltd.)
·Chenodeoxycholic acid (from Tokyo Chemical Industry Co., Ltd.)
·Hyodeoxycholic acid (from Tokyo Chemical Industry Co., Ltd.)
·Sodium glycodeoxycholate (from Nacalai Tesque Inc.)
·Monoammonium glycyrrhizinate (from Tokyo Chemical Industry Co., Ltd.)
·EYPC (non-hydrogenated egg yolk phosphatidylcholine: from NOF Corporation, COATSOME NC-50)
·DDPC (1,2-didecanoyl-sn-glycero-3-phosphatidylcholine: from NOF Corporation, COATSOME MC-1010)
·DLPC (1,2-dilauroyl-sn-glycero-3-phosphatidylcholine: from NOF Corporation, COATSOME MC-1212)
·Polyoxyethylene sorbitan monofatty acid ester (Tween 20, 80: from Tokyo Chemical Industry Col, Ltd.)
·Sorbitan fatty acid ester (SPAN 80: from Nacalai Tesque Inc., sorbitan monooleate)
·Polyoxyethylene castor oil (from Wako Pure Chemical Industries, Ltd., Polyoxyethylene 10 castor oil)
·Tocopherol (from Nacalai Tesque Inc., DL-α-tocopherol)

(2) Substance having stimulus that activates natural immunity, etc.

·CpG-DNA (CpG-ODN: from InvivoGen, ODN-2395)
·Tocopherol acetate (DL-$\alpha$-tocopherol acetate: from Nacalai Tesque Inc.)
·Aluminum hydroxide (from InvivoGen, Alhydrogel 2%)
·Aluminum phosphate (from Alpha Diagnostic International, Adjuphos)
·Aluminum potassium sulfate (from InvivoGen, alum crystals)

(3) Solvent, etc.

·Water for injection (from Otsuka Pharmaceutical Co., Ltd.)
·MES-Na (from Merck Ltd.)
·Sodium chloride (from Kanto Chemical Co., Inc.)
·PBS Tablets (Phosphate buffered saline: from Takara Bio Inc.)
·Methanol (from Nacalai Tesque Inc.)
·Ethanol (from Kanto Chemical Co., Inc.)
·Chloroform (from Wako Pure Chemical Industries, Ltd.)
·Aqueous sodium hydroxide solution (0.1 mol/L: from Nacalai Tesque Inc.)
·Hydrochloric acid (0.1 mol/L, 1 mol/L: from Nacalai Tesque Inc.)
·OVA peptide: SIINFEKL (outsourced for synthesis by PH Japan Co., Ltd.) (This may be simply referred to as "peptide" hereinafter.)
·OVA protein (Ovalbumin: from Wako Pure Chemical Industries, Inc., ovalbumin low endotoxin) (This may be simply referred to as "OVA" hereinafter.)

(4) Culture medium, etc.

·RPMI (from Nacalai Tesque Inc., RPMI 1640 culture medium (liquid))
·Penicillin-Streptamycin Mixed Solution (from Nacalai Tesque Inc.)
·FBS (Fetal Bovine Serum, Centified, Heat Inactivated, US Origin: from Gibco)
·RBC lysis buffer (from Santa Cruz Biotechnology): erythrocyte hemolytic buffer
·HBSS (Habk's Balanced Salt Solution: from Nacalai Tesque Inc.)
·D-PBS (Dulbecco's Phosphate Buffered Saline: from Nacalai Tesque Inc.)

(5) Other reagents

·Phospholipid C-Test wako (from Wako Pure Chemical Industries, Ltd.)
·Pyranine (from Tokyo Chemical Industry Co., Ltd.)
·DPX (p-xylene-bis-pyridinium bromide: from Molecular probes Inc.)
·Triton-X100 (from Wako Pure Chemical Industries, Ltd.)
·NBD-PE (1,2-dioleoyl-sn-glycero-3-phosphoethanolamine-N(7-nitro-2-1,3-benzoxadiazol-4-yl)ammonium: from Avanti Polar Lipids, Inc.)
·Rh-PE (1,2-dioleyl-sn-glycero-3-phosphoethanolamine-N-(lissamine rhodamine B sulfonyl)ammonium: from Avanti Polar Lipids, Inc.): fluorescence labeling lipid
·Mouse IFN$\gamma$ ELISPOT Set (from BD Biosciences, Inc.)
·AEC Substrate Set (from BD Biosciences, Inc.)
·Sodium hydrogen carbonate (from Wako Pure Chemical Industries, Inc.)
·Sodium carbonate (from Wako Pure Chemical Industries, Inc.)
·Secondary antibody (anti-mouse IgG HRP conjugate, from R&D systems, Inc.)
·Albumin (Albumin from bovine serum, from Sigma Corporation)
·Color development kit for peroxidase (from Sumitomo Bakelite Co., Ltd.)

(6) Animals
C57BL/6N mouse, female, (six to eight weeks old), was bought from Japan S.L.C, Inc. Experiments were carried out according to the guideline for animal experiments drafted by Terumo Corporation.

<Apparatus used>

Ultrasonic irradiator: USC-J
Spectrophotometer: FP-6500
UV-vis spectrophotometer: UV-3600
$CO_2$ incubator: MCO20AIC

<Cell culture>

Cell culture was carried out using an incubator (MCO20AIC) set to 5% $CO_2$ and 37°C.

<Sample preparation, etc.>

(MES Buffer)

Prepared from MES (25 mM) and NaCl (125 mM). The MES Buffer has a pH value of 7.4, unless otherwise specified.

(PBS)

PBS was prepared by dissolving 10 pieces of PBS Tablets (from Takara Bio Inc.) in 1,000 mL of distilled water. Note that pH was 7.35 to 7.65. Alternatively, purchased D-PBS was used as the PBS.

(RPMI medium)

Penicillin (100 unit/mL) and streptomycin (100 mg/mL) were added as antibiotics. Besides, FBS was additionally added, as required, to obtain RPMI medium containing 10% serum.

(Primary solution of candidate of natural immunity-activating substance (this substance may be referred to as candidate substance hereinafter))

·Primary solution of tocopherol
10 mg of tocopherol was weighed and dissolved in 1.0 mL of ethanol. The resulting solution was further 10-fold diluted with PBS, to prepare a primary solution of tocopherol.
·Primary solution of tocopherol acetate
10 mg of tocopherol acetate was weighed and dissolved in 1.0 mL of ethanol. The resulting solution was further 10-fold diluted with PBS, to prepare a primary solution of tocopherol acetate.
·Primary solution of alum
Aluminum hydroxide, aluminum phosphate, and aluminum potassium sulfate were used as alum.

In the case where aluminum hydroxide was used as alum and where the final concentration was 100 pg/100 μL, the raw liquid of the reagent purchased (aluminum concentration (as aluminum metal): approximately 10 mg/mL) was used as a primary solution of alum, for preparation of the adjuvant composition. In the case where the final concentration was lower than 100 pg/100 μL, the raw liquid of the reagent purchased was diluted with PBS to obtain a primary solution of alum, which was used for preparation of the adjuvant composition. Specifically, in the case where the final concentration was 10 pg/100 μL, the raw liquid of the reagent purchased was 10-fold diluted with PBS, before being used for preparation of the adjuvant composition. In the cases where the final concentration was 1 pg/100 μL and 0.1 pg/100 μL, 100-fold dilution with PBS and 1,000-fold dilution with PBS were conducted, respectively, to obtain primary solutions of alum, which were used for preparation of adjuvant compositions.
In the case where aluminum phosphate was used as alum, a raw liquid of the reagent purchased and a liquid obtained by diluting the raw liquid with PBS were used as primary solutions of alum, for preparation of adjuvant compositions, like in the case of aluminum hydroxide.
10 mg of aluminum potassium sulfate was weighed and dissolved in 1 mL of water for injection. The resulting solution was handled in similar manner to the raw liquid of the reagent purchased in the case of aluminum hydroxide, to be used for preparation of adjuvant compositions.

(Preparation of pH-sensitive carrier)

Production of Bangham method

1,000 nmol of an amphipathic substance dissolved in methanol (or chloroform) and a pH-sensitive compound dissolved in methanol (or chloroform) were mixed in an 10-mL eggplant-shaped flask, and the solvent was volatilized, to obtain a thin film. To the thus formed thin film, 0.5 mL of MES Buffer (in the case of the release test and the membrane-fusion test) or 0.5 mL of PBS (in the case where immunization of mouse) was added, and dispersion was conducted using an ultrasonic irradiator, to prepare a dispersion (solution) of the pH-sensitive carrier. Besides, at the time of use, the dispersion (solution) was diluted with MES Buffer or PBS, while taking the total amount into consideration.

Production by mixing

A predetermined amount of a pH-sensitive compound was weighed, and was dissolved in MES Buffer or PBS, to prepare a solution of the pH-sensitive compound. Next, a predetermined amount of an amphipathic substance was weighed, the resulting solution was added to and mixed with the previously prepared solution of the pH-sensitive compound, to thereby prepare a dispersion (solution) of a pH-sensitive carrier. The operation was carried out at room temperature. Specifically, in the case of a pH-sensitive carrier composed of 1,600 nmol of deoxycholic acid per 1,000 nmol of DLPC, 13.8 mg of sodium deoxycholate monohydrate was weighed, and was dissolved in 10 mL of MES Buffer or PBS. Next, 12.4 mg of DLPC was weighed, and was added to and mixed with the previously prepared deoxycholic acid solution, to thereby prepare a dispersion of a pH-sensitive carrier. Besides, at the time of use, the dispersion (solution) was diluted with MES Buffer or PBS, while taking the total amount into consideration.

Note that the ratio between the amphipathic substance and the pH-sensitive compound was adjusted to a desired ratio. In addition, in the case of using a plurality of amphipathic substances, adjustment was made such that their total amount was a desired molar number (1,000 nmol). Besides, the amount of the pH-sensitive compound used indicated in Examples and the explanation of figures is the amount per 100 nmol of the amphipathic substance.

(Preparation of adjuvant composition and vaccine composition)

A primary solution of a candidate of a natural immunity-activating substance (hereinafter referred to also as candidate substance) was added to the prepared dispersion (solution) of the pH-sensitive carrier, followed by mixing. For adjusting concentration, additional MES Buffer or PBS was added to the admixture, followed by mixing, to prepare an adjuvant composition.

Besides, PBS with a predetermined amount of OVA dissolved therein was added to the thus obtained adjuvant composition, followed by mixing, to prepare a vaccine composition.

In other experiments than the experiment of evaluation (4), the pH-sensitive carrier, the adjuvant composition, and the vaccine composition were adjusted so as to obtain an amphipathic substance concentration of 100 nmol/100 $\mu$L. In the experiment of evaluation (4), the dispersion (solution) of the pH-sensitive carrier was diluted, before being used. Finally, the vaccine composition was prepared in such a manner as to obtain an amphipathic substance concentration of 100 nmol/100 $\mu$L to 0.1 nmol/100 $\mu$L.

In the case of using CpG-DNA (CpG-ODN), 200 $\mu$g of CpG-ODN was dissolved in 200 $\mu$L of water for injection, to obtain a primary solution of a candidate substance, which was used for preparation of the adjuvant composition.

(Preparation of comparative samples)

Comparative samples were prepared by using an antigen alone or using a mixture of an antigen and a candidate substance (referred to also as candidate substance alone). In addition, in the release test, a dispersion of an amphipathic substance alone and a dispersion of a pH-sensitive carrier alone were also prepared.

<Method for measurement>

(Release test: Measurement of leakage)

The leakage was evaluated according to the method described in K. Kono et al., Bioconjugate Chem. 2008 19 1040-1048. The evaluation was performed using EYPC liposomes which contain Pyranine as a fluorescent substance and DPX as a quencher.

3,000 nmol of EYPC dissolved in chloroform was measured and placed in a 10-mL eggplant-shaped flask, and the EYPC was made into thin film by means of a rotary evaporator. To the flask was added 500 $\mu$L of a Pyranine solution (Pyranine: 35 mM, DPX: 50 mM, MES: 25 mM, pH 7.4), followed by dispersion using an ultrasonic irradiator (USC-J), and then the resulting dispersion was passed through a polycarbonate film having a pore diameter of 100 nm by use of an extruder, so as to make the particle diameter uniform. The resulting dispersion underwent substitution of the outer aqueous layer using MES Buffer and G100 column, to obtain a dispersion of EYPC liposomes embracing the fluorescent substance. The concentration of the phospholipid was determined using phospholipid C-test Wako, and it was adjusted with MES Buffer so that phospholipid became 1.0 mmol/L.

20 μL of the dispersion of EYPC liposomes adjusted in concentration and 20 μL of an evaluation sample dispersion were added to 2960 μL of pH-adjusted MES Buffer, and the resulting mixture was subjected to incubation at 37°C for 90 or 30 minutes (Values resulting from incubation for 90 minutes are adopted in Examples, unless otherwise specified.). Fluorescence at Ex461 nm and Em512 nm was observed by use of a spectrophotometer FP-6500 to monitor the leakage.

Note that the leakage was calculated assuming that the value in the case of using the dispersion alone of EYPC liposomes is 0% and the value in the case of adding 30 μL of 10-fold diluted Triton-X100 is 100%. Specifically, the leakage was calculated according to the following formula. Note that in the following formula, L represents the intensity of fluorescence measured, $L_0$ represents the intensity of fluorescence of only the dispersion of EYPC liposomes embracing the fluorescent substance, and $L_{100}$ represents the intensity of fluorescence in the case of adding Triton-X100.

[Math. 3]

$$\text{Leakage (\%)} = \frac{(L - L_0)}{(L_{100} - L_0)} \times 100$$

(Membrane-fusion test: Measurement of Fusion (membrane-fusion))

The Fusion (membrane-fusion) was evaluated according to the method described in K. Kono et al., Biomaterials 2008 29 4029-4036, by use of FRET (Fluorescence Resonance Energy Transfer). The fluorescence labeling was conducted using NBD-PE and Rh-PE.

Thin film of EYPC (EYPC 1,000 nmol) containing 0.6 mol% of NBD-PE and Rh-PE relative to EYPC was produced. The thin film was dispersed together with 1.0 mL of MES Buffer added thereto, by use of an ultrasonic irradiator (USC-J). The resulting dispersion was passed through a polycarbonate film having a pore diameter of 100 nm by use of an extruder, to obtain a dispersion of EYPC liposomes with double fluorescence labeling.

20 μL of the dispersion of EYPC liposomes with double fluorescence labeling and 20 μL of the evaluation sample dispersion were added to 2960 μL of the pH-adjusted MES Buffer, followed by incubation at 37°C for 60 minutes. The resulting liquid was measured for fluorescence spectrum at 500 to 620 nm by using a spectrophotometer (FP-6500) with excitation light at 450 nm. There was obtained the ratio of the fluorescence intensity at 520 nm and that at 580 nm.

The fusion rate was calculated assuming that the fluorescence intensity ratio in the case where the dispersion of double fluorescence labeled EYPC liposomes obtained above and the amphipathic substance are incubated is 0% and that the fluorescence intensity ratio in the case where the dispersion of double fluorescence labeled EYPC liposomes and the evaluation sample dispersion are subjected to a methanol treatment is 100%. Note that the methanol treatment consists of dissolving the dispersion of double fluorescence labeled EYPC liposomes and the evaluation sample dispersion in methanol, making the resulting solution into thin film by use of a rotary evaporator, and dispersing the thin film into 3.0 mL of MES Buffer by use of the ultrasonic irradiator.

Specifically, the fusion rate was calculated according to the following formula. Note that in the following formula, R represents the fluorescence intensity ratio measured, $R_0$ represents the fluorescence intensity ratio in the case where the dispersion of the double fluorescence labeled EYPC liposomes and the amphipathic substance are incubated, and $R_{100}$ represents the fluorescence intensity ratio obtained when the dispersion of double fluorescence labeled EYPC liposomes and the evaluation sample dispersion are subjected to the methanol treatment.

[Math. 4]

$$\text{Fusion rate (\%)} = \frac{(R - R_0)}{(R_{100} - R_0)} \times 100$$

(Immunization of mouse)

Administration was performed under anesthesia. In the case of subcutaneous administration, it is carried out by subcutaneous injection, 100 $\mu$L/head, at one position on the back. The dose of alum was 0 to 100 $\mu$g/head, and that of the candidate of the natural immunity-activating substance was 10 $\mu$g/head. As for the pH-sensitive carrier, a fundamental dose concentration for the amphipathic substance was 100 nmol/head, that for the pH-sensitive compound was 10 to 640 nmol/head, and part thereof was administered in diluted conditions. As the antigen, OVA protein or OVA peptide was used, the dose being 80 $\mu$g/head. Besides, in the case of intracutaneous administration, intracutaneous injection, 20 $\mu$L/head, was performed at one position on the back using the administration liquid similarly in the case of subcutaneous administration. In the case of evaluating cell-mediated immunity, administration was performed once and assay was conducted seven days after the administration. In the case of evaluating humoral immunity, administration was performed twice. The second administration was conducted 14 days after the first administration, and assay was performed 14 days after the second administration.

(Preparation of dispersion of cells from mouse's spleen)

The mouse was euthanized on the seventh day after the last administration and had its spleen extracted. After addition of 3.0 mL of RPMI medium containing 10% serum, the spleen was treated by the BD Falcon cell strainer, to obtain a suspension of cells. The cells were subjected to hemolysis with RBC lysis buffer, followed by washing with RPMI medium containing 10% serum. The cells were dispersed into RPMI medium containing 10% serum, after which the number of cells was counted, and a dispersion of spleen cells was obtained.

(ELIspot method)

The ELIspot method was carried out by using the Mouse IFN$\gamma$ ELISPOT Set. One day before cell inoculation, the 96-well ELIspot plate was allowed to adsorb the detection antibody attached to the kit, to make the plate. The thus made plate was washed with RPMI medium containing 10% serum, after which 200 $\mu$L of 10% serum-containing RPMI medium was added thereto, and the plate was left to stand at 37°C for two hours for blocking. The plate was washed with 10% serum-containing RPMI medium. In the case where the antigen undergoes restimulation, 100 $\mu$L of 10% serum-containing RPMI medium containing 40 ug/mL of OVA peptide was added to the plate. In the case where the antigen does not undergo restimulation, 100 $\mu$L of 10% serum-containing RPMI medium was added to the plate. The plate was inoculated with the dispersion of spleen cells so that there existed a predetermined number of cells. Finally, 10% serum-containing RPMI medium was added so that the total amount per well was adjusted to 200 $\mu$L. Thereafter, cultivation was performed for two nights, and coloration of the plate was conducted.

The coloration of the plate was carried out according to the protocol described in the Mouse IFN$\gamma$ ELISPOT Set and the AEC Substrate Set.

(Measurement of antibody titer)

Partial blood collection was conducted one day before the second administration, and the antibody titer after administration once was examined. In addition, blood collection was performed 14 days after the second administration, and the antibody titer after administration twice was examined. The collected blood was left to stand at room temperature for two hours, followed by centrifugation, to obtain serum. Block buffer was prepared by dissolving 5 g of albumin in 500 mL of PBS. Coating buffer was prepared by dissolving 1.47 g of sodium hydrogen carbonate and 0.80 g of sodium carbonate in 500 mL of water. Washing of the plate was performed using a liquid obtained by adding 2.5 mL of Tween 20 to 500 mL of PBS.

In the coating buffer was dissolved OVA protein, and the resulting solution was added to the 96-well plate such that the amount was 0.1 $\mu$g/well (100 $\mu$L). After standing at 37°C for two hours, the coating buffer was replaced by 300 $\mu$L of Block buffer, followed by standing overnight at 4°C. After the plate was washed, 100 $\mu$L of serum diluted by a predetermined factor was added to each well, followed by standing at 37°C for two hours. After the plate was washed, a 100 $\mu$L of secondary antigen solution diluted 1,000 fold with Block buffer was added to each well, followed by standing at 37°C for two hours. After the plate was washed, color development was conducted using the coloring kit for peroxidase, and the antibody titer was obtained.

[Evaluation]

Various evaluations were performed by using the dispersions prepared according to the above-mentioned methods.

(1) Selection of natural immunity-activating substance suitable for joint use with pH-sensitive carrier

Selection of natural immunity-activating substances that has a high effect of inducing cell-mediated immunity when used jointly with the pH-sensitive carrier was performed. Taking into account the availability and actual results of injection, tocopherol, tocopherol acetate, and alum (aluminum hydroxide) were used as candidates of natural immunity-activating substance (hereinafter referred to also as candidate substances), and they were examined for the effect of inducing cell-mediated immunity when used jointly with the pH-sensitive carrier.

As the pH-sensitive carrier, a complex of 100 nmol of DLPC and 160 nmol of deoxycholic acid, per mouse, was used. The candidate substances were all used in an amount of 10 μg per mouse, and OVA protein was used as the antigen in an amount of 80 μg per mouse. Administration liquids were prepared by sequentially adding the dispersion (solution) of the pH-sensitive carrier, the candidate substance, and the antigen in this order to PBS for controlling a dose, and mixing them. Note that the administration liquids were prepared in such a manner that the dispersion (solution) of the pH-sensitive carrier, the candidate substance, and the antigen were present in predetermined concentrations in 100 μL of the liquid. The dose was 100 μL per mouse, and the administration route was subcutaneous. Induction of cell-mediated immunity was evaluated according to the ELIspot method described hereinabove.

Besides, as a comparative sample, the antigen alone and a mixture of the antigen and a candidate substance were used; as the administration liquid, a liquid prepared by mixing PBS with the antigen or with the antigen and the candidate substance was used, and was administered in a dose of 100 μL per mouse.

In addition, induction of cell-mediated immunity (CTL) by the ELIspot method was compared with that for a control (a mixture of the antigen and the aluminum-containing substance) by visual observation, and was evaluated according to the following criterion.

∘: Induction of cell-mediated immunity is observed clearly as compared to the control
Δ: Induction of cell-mediated immunity is observed slightly as compared to the control
×: Induction of cell-mediated immunity is comparable to or lower than that of the control

As a result of evaluation, naturally, use of the antigen alone did not lead to spot formation, and did not induce cell-mediated immunity (FIG. 2(A)). Besides, uses of tocopherol and tocopherol acetates as comparative samples failed to cause spot formation, like the use of the antigen alone, and did not induce cell-mediated immunity (FIGS. 2(B) and (C)). Further, since alum is widely known to be unable to induce cell-mediated immunity, spot formation in FIG. 2(D) was determined to be the absence of induction of cell-mediated immunity. Therefore, it is considered that cell-mediated immunity is induced when the number of spots formed is greater than that in FIG. 2(D).

Joint use of tocopherol with the pH-sensitive carrier and joint use of tocopherol acetate with the pH-sensitive carrier led to the formation of the number of spots comparable to that by alum alone (the antigen + alum in FIG. 2(D)), and thus could not induce cell-mediated immunity (FIGS. 2(E) and (F)). On the other hand, joint use of alum with the pH-sensitive carrier led to formation of a much greater number of spots as compared to the number of spots formed by alum alone (FIG. 2(G)). It is thus verified that the joint use of alum with the pH-sensitive carrier leads to induction of cell-mediated immunity. This result showed that alum is a natural immunity-stimulating (activating) substance suitable for joint use with the pH-sensitive carrier.

(2) Comparison with CpG-ODN and evaluation of antigen specificity

CpG-ODN is a synthetic oligonucleotide having a specific base sequence, and is a reagent which has been reported to induce cell-mediated immunity. By using this reagent as a positive control, the level of activity to induce cell-mediated immunity by joint use of alum with the pH-sensitive carrier was evaluated. CpG-ODN was used in an amount of 10 μg per mouse, and the other experimental conditions were all the same as those in (1). The evaluation was conducted using the same plate as that of ELIspot in (1).

The number of spots formed obtained by joint use of alum with the pH-sensitive carrier was greater than that obtained by CpG-ODN (FIGS. 3(B) and (C)). Showing a high activity as compared to CpG-ODN which is a generally known reagent, the joint use of alum with the pH-sensitive carrier has been revealed to enable high induction of cell-mediated immunity.

In addition, the fact that the induced immunity is antigen-specific is very important from the viewpoint of practical use. In view of this, the cell-mediated immunity induced by the joint use of alum with the pH-sensitive carrier was evaluated for antigen specificity. The evaluation was conducted according to the above-described ELIspot method in which restimulation is not applied. Specifically, a dispersion of spleen cells recovered from an immunized animal was inoculated to the ELIspot plate, and spot formation by IFNγ production cells, without addition of peptide of SIINFEKL, was performed. As a result, spot formation was not observed at all, and the spot formation obtained by the joint use of alum with the pH-sensitive carrier in FIG. 3(B) disappeared completely (FIG. 3(D)). Thus, it was shown that the cell-mediated immunity induced by the joint use of alum with the pH-sensitive carrier is surely antigen-specific. In addition, the induced cells showed reaction with class I peptide of SIINFEKL, and, thus, it was suggested that the induced cells were CTL.

(3) In regard of amount of alum necessary for inducing cell-mediated immunity

It was made clear that alum (aluminum hydroxide) induces cell-mediated immunity when used jointly with the pH-sensitive carrier. In view of this, the amount of alum needed for inducing cell-mediated immunity was examined. Aluminum hydroxide was used as alum, and a complex of DLPC and deoxycholic acid was used as the pH-sensitive carrier (100 nmol of DLPC and 160 nmol of deoxycholic acid per mouse). The alum was used in an amount of 100 to 0.1 $\mu$g per mouse, and OVA protein was used as the antigen in an amount of 80 $\mu$g per mouse. Administration liquids were prepared by sequentially adding PBS for controlling a dose, alum, and the antigen in this order to a dispersion (solution) of the pH-sensitive carrier, and mixing them. The dose was 100 $\mu$L per mouse, and the administration route was subcutaneous. Induction of cell-mediated immunity was evaluated according to the above-described ELIspot method. Note that "alum alone" refers to a mixture of the antigen and alum.

As a result of the evaluation, a vaccine composition containing 100 to 1 $\mu$g of alum (the alum concentration in the solution was 100 pg/100 $\mu$L to 1 pg/100 $\mu$L) (in the figure, antigen + alum + pH-sensitive carrier) led to formation of the number of spots greater than that by alum alone (in the figure, antigen + alum) (FIGS. 4(B), (C), (D), and (E)). On the other hand, in the case where alum was used in an amount of 0.1 $\mu$g, the number of spots formed was comparable to that by alum alone (FIGS. 4(B) and (F)). From these results, an effect of inducing cell-mediated immunity was confirmed when the amount of alum was 1 to 100 $\mu$g (the alum concentration in the solution was 1 ug/100 $\mu$L to 100 ug/100 $\mu$L) per 100 nmol of DLPC. Thus, it was suggested that cell-mediated immunity is induced when the amount of alum is 1 to 100 $\mu$g (alum concentration: 1 gg/100 $\mu$L to 100 ug/100 $\mu$L) per 100 nmol of the amphipathic substance.

(4) In regard of amount of pH-sensitive carrier necessary for inducing cell-mediated immunity

Next, the amount of the pH-sensitive carrier needed to induce cell-mediated immunity was examined. Administration liquids were prepared by using aluminum hydroxide in an amount of 10 ug per mouse, and using dispersions of the pH-sensitive carried diluted by various factors. A complex composed of 160 nmol of deoxycholic acid per 100 nmol of DLPC was used as the pH-sensitive carrier. The other conditions were similarly to those in (1) and (3).

As a result of the evaluation, the number of spots formed by use of the prepared administration liquid was greater than that by alum alone, in the case where the dilution factor of the pH-sensitive carrier was 1 to 100 (fold) (FIGS. 5(B), (C), (D), and (E)). On the other hand, the 1,000-fold diluted administration liquid led to formation of the number of spots slightly greater than that by alum alone (FIGS. 5(B) and (F)). From these results, it was confirmed that the administration liquid prepared such that the DLPC constituting the pH-sensitive carrier was in a concentration of 1 to 1,000 nmol/100 $\mu$L and deoxycholic acid was in a concentration of 1.6 to 160 nmol/1,000 $\mu$L induces cell-mediated immunity. Thus, it was suggested that the vaccine composition induces cell-mediated immunity in the case where the concentration of the amphipathic substance constituting the pH-sensitive carrier is 1 to 1,000 nmol/100 $\mu$L.

(5) In regard of induction of humoral immunity

Induction of humoral immunity by joint use of alum and the pH-sensitive carrier was verified. The induction of humoral immunity was evaluated by IgG antigen titer. As for dose per administration, 10 $\mu$g of aluminum hydroxide was used as alum, while a complex composed of 100 nmol of DLPC and 160 nmol of deoxycholic acid was used as the pH-sensitive carrier. OVA protein in an amount of 80 $\mu$g was used as the antigen. The administration liquids were prepared by sequentially adding PBS for controlling a dose, alum, and the antigen in this order to a dispersion (solution) of the pH-sensitive carrier, and mixing them. The dose was 100 $\mu$L per mouse, and the administration route was subcutaneous. For evaluation of humoral immunity, administration was performed twice. The evaluation of antibody titer was carried out according to the above-mentioned measurement of antibody titer.

As a result of the evaluation, use of alum alone showed a highest IgG antibody titer, and joint use of alum with the pH-sensitive carrier showed a second highest antibody titer (FIG. 6, administered twice). In addition, the joint use of alum and the pH-sensitive carrier showed a high IgG antibody titer as compared to the groups of using PBS or the antigen alone (FIG. 6, administered twice). From these results, it was shown that the vaccine composition obtained by joint use of alum and the pH-sensitive carrier induces humoral immunity.

It was made clear that the vaccine composition prepared by joint use of alum and the pH-sensitive carrier is capable of inducing and enhancing both cell-mediated immunity and humoral immunity. Therefore, the adjuvant composition according to the present invention can be used as an excellent adjuvant.

(6) In regard of the kind of alum

Herein the term "alum" is used as a generic name of aluminum-containing adjuvants. The kinds of alum include aluminum hydroxide, aluminum phosphate, aluminum potassium sulfate, and aluminum chloride. Among these, the ones which are available and capable of inducing cell-mediated immunity when used jointly with the pH-sensitive carrier were examined.

In this Example, aluminum phosphate and aluminum potassium sulfate were used as alum, while a complex of DLPC and deoxycholic acid (100 nmol of DLPC and 160 nmol of deoxycholic acid per mouse) was used as the pH-sensitive carrier. With reference to Example of (3), alum was used in an amount of 1 to 0.1 $\mu$g per mouse, and OVA protein was used as the antigen in an amount of 80 $\mu$g per mouse. Administration liquids were prepared by sequentially adding PBS for controlling a dose, alum, and the antigen in this order to a dispersion (solution) of the pH-sensitive carrier, and mixing them. The dose was 100 $\mu$L per mouse, and the administration route was subcutaneous. The induction of cell-mediated immunity was evaluated according to the above-described ELIspot method.

As a result of the evaluation, it was found that the use of the mixture of the antigen with aluminum phosphate and the use of the mixture of the antigen with aluminum potassium sulfate led to little observable spot formation, and could not induce cell-mediated immunity (FIGS. 7(A) and (D)). The vaccine composition prepared using 1 $\mu$g of aluminum phosphate led to formation of spots the number of which was greater than that by the mixture of the antigen with aluminum phosphate, and induced cell-mediated immunity (FIG. 7(B)). On the other hand, the vaccine composition prepared using 0.1 $\mu$g of aluminum phosphate led to spot formation similar to that by the mixture of the antigen with aluminum phosphate, and did not induce cell-mediated immunity (FIG. 7(C)). As to the minimum amount of alum needed for inducing cell-mediated immunity, aluminum phosphate showed results similarly to those of aluminum hydroxide; for this reason, aluminum phosphate is considered to induce cell-mediated immunity when the amount of alum is 1 to 100 $\mu$g (alum concentration: 1 gg/100 $\mu$L to 100 ug/100 $\mu$L) per 100 nmol of the amphipathic substance, in the manner similarly to the case of aluminum hydroxide.

In addition, the vaccine composition prepared using 1 $\mu$g of aluminum potassium sulfate showed formation of spots the number of which was slightly greater than that by the mixture of the antigen and aluminum potassium sulfate, which suggests that cell-mediated immunity was induced (FIG. 7(E)).

From these results, it was found that aluminum hydroxide and aluminum phosphate are higher than aluminum potassium sulfate in the effect.

It was made clear that aluminum hydroxide and aluminum phosphate are particularly useful as a constituent of a vaccine composition for inducing cell-mediated immunity when used jointly with the pH-sensitive carrier.

(7) In regard of influence of alum on function of pH-sensitive carrier

(7-1) In regard of influences of the kind of alum and the amount of alum on effect of promoting membrane-disruption function

The effect promoting the membrane-disruption function and the effect of promoting the membrane-fusion function of the pH-sensitive carrier are important functions for disrupting the endosome membrane of cells and promoting cross-presentation. In addition, alum is known to adsorb various substances. Therefore, influence of mixture of alum and the pH-sensitive carrier on the function of the pH-sensitive carrier was examined.

First, the influence on the effect of promoting the membrane-disruption function was examined. A dispersion (solution) of the pH-sensitive carrier and alum were mixed to prepare evaluation samples such that the total amount of the evaluation sample was 100 $\mu$L. After incubating the evaluation samples overnight, part (20 $\mu$L) of each evaluation sample was put to a release test. The release test was conducted according to the above-described release test. In 100 $\mu$L, a complex of 100 nmol of DLPC and 160 nmol of deoxycholic acid was used as the pH-sensitive carrier, while aluminum hydroxide, aluminum phosphate and aluminum potassium sulfate were used as alum in an amount of 10 $\mu$g.

Deoxycholic acid used alone showed a leakage of not more than 1% at pH 7.4, and a leakage of approximately 15% at pH 5.0; the pH-sensitive carrier not mixed with alum showed a leakage of not more than 1% at pH 7.4 and a leakage of approximately 60% at pH 5.0 (FIG. 8(A)). The leakage shown by DLPC alone was not more than 2% at pH 5.0 (FIG. 10(A)), so that the pH-sensitive carrier surely had an effect of promoting the membrane-disruption function. In addition, the pH-sensitive carrier mixed with any alum of aluminum hydroxide, aluminum phosphate, and aluminum potassium sulfate caused little release at pH 7.4, and caused release of approximately 60% at pH 5.0 (FIG. 8(A)). Thus, when mixed with any alum, the pH-sensitive carrier satisfied the formula (1) and formula (2), which shows that the mixed pH-sensitive carrier surely has the effect of promoting the membrane-disruption function. Besides, in the case of any alum, the pH-sensitive carrier mixed with alum showed a leakage comparable to that shown by the pH-sensitive carrier not mixed with alum, at pH 7.4 and 5.0. Thus, it was made clear that mixing with alum does not influence the effect of promoting the membrane-disruption function of the pH-sensitive carrier.

Subsequently, influence of the amount of alum mixed was examined. Alum consisting of each of aluminum hydroxide, aluminum phosphate, and aluminum potassium sulfate was mixed with the pH-sensitive carrier composed of 100 nmol of DLPC and 160 nmol of deoxycholic acid, in an amount of 0 to 100 $\mu$g (total amount: 100 $\mu$L). The resulting solutions were incubated overnight, and put to the release test. Note that the solution prepared using 100 $\mu$g of aluminum potassium sulfate was low in pH, and, hence, was not to put to measurement. As a result, in the case of any alum and in any addition amount, the leakage at pH 5.0 was approximately 60%, and no change was generated as compared to the case of the pH-sensitive carrier not mixed with alum (FIG. 8(B)). Therefore, it was suggested that alum, irrespective of the kind thereof and irrespective of the addition amount thereof, has no influence on the effect of promoting the membrane-disruption function of the pH-sensitive carrier.

(7-2) Influence of alum on effect of promoting membrane-fusion function

Subsequently, influence of alum on the effect of promoting the membrane-fusion function was examined. FIG. 9 depicts the results of examination of the effect of promoting the membrane-disruption function of the pH-sensitive carrier in the cases of mixture with various kinds of alum. As the amphipathic substance was used 100 nmol of DLPC, as the pH-sensitive compound was used 160 nmol of deoxycholic acid, and alum was used in an amount of 10 $\mu$g. A dispersion (solution) of the pH-sensitive carrier and alum were mixed so that the total amount was 100 $\mu$L. The resulting mixed solutions were incubated overnight, and part of each of the mixed solutions was evaluated by a membrane fusion test. The membrane fusion test was conducted according to the above-described membrane fusion test.

The pH-sensitive carrier, mixed with any alum, showed a fusion rate as high as approximately 70% at pH 5.0, and satisfied the formula (3) (FIG. 9). Thus, the pH-sensitive carrier mixed with alum surely had an effect of promoting the membrane-fusion function. In addition, in the cases of any alum, the pH-sensitive carrier mixed with alum showed fusion rates comparable to those shown by the pH-sensitive carrier not mixed with alum, at pH 7.4 and 5.0 (FIG. 9). Therefore, it was confirmed that mixing with alum produces no influence on the effect of promoting the membrane-fusion function of the pH-sensitive carrier.

The pH-sensitive carrier showed similar effect of promoting the membrane-disruption function and similar effect of promoting the membrane-fusion function, irrespectively of mixing with alum. Thus, it was made clear that mixing with alum produces no influence of the function of the pH-sensitive carrier.

(7-3) Influence of alum in pH-sensitive carriers containing deoxycholic acid in various amounts

Next, influences of alum on the function of the pH-sensitive carrier in the case where the composition of the pH-sensitive carrier was varied were examined. As alum was used 10 $\mu$g of aluminum hydroxide, and complexes composed of 100 nmol of DLPC and various amounts of deoxycholic acid were used as the pH-sensitive carrier (total amount: 100 $\mu$L). Like in (7-1), dispersions (solutions) of the pH-sensitive carriers and alum were mixed. The resulting mixed solutions were incubated overnight, and part of each of the mixed solutions was put to the release test. The release test was carried out according to the above-described release test.

At pH 5.0, the pH-sensitive carriers mixed with alum, at any amount of deoxycholic acid, showed leakage values comparable to those shown by the pH-sensitive carrier not mixed with alum (FIGS. 10(A) and (B)). In other words, it was shown that in the case of the pH-sensitive carriers composed of 100 nmol of DLPC and 10 to 640 nmol of the pH-sensitive compound, mixing with alum produced no influence on the function of the pH-sensitive carriers. It was suggested that in the cases of the pH-sensitive carriers, containing any amount of the pH-sensitive compound, mixing with alum produces no influence on the function of the pH-sensitive carriers.

In addition, the pH-sensitive carriers mixed with alum, with the amount of the pH-sensitive compound being at any value in the range of 10 to 640 nmol, satisfied the formula (1) and formula (2), and, thus, surely had the effect of promoting the membrane-disruption function. These pH-sensitive carriers are considered to induce cell-mediated immunity by being mixed with alum.

(7-4) Influence of alum in pH-sensitive carriers composed of various pH-sensitive compounds

Further, influence of alum on the functions of pH-sensitive carriers composed of various pH-sensitive compounds was examined. As alum was used 10 $\mu$g of aluminum hydroxide, and complexes composed of 100 nmol of DLPC and 160 nmol each of various pH-sensitive compounds were used as the pH-sensitive carrier (total amount: 100 $\mu$L). Like in (7-1), the pH-sensitive carriers and alum were mixed, the resulting mixed solutions were incubated overnight, and part of each of the mixed solutions was evaluated by the release test. The release test was carried out according to the above-described release test. The leakage shown by DLPC (amphipathic substance) used alone was not more than 2.0% at pH 5.0.

In the cases of the pH-sensitive carriers prepared by use of any pH-sensitive compound, the pH-sensitive carriers mixed with alum showed equivalent leakage values comparable to those shown by the pH-sensitive carriers not mixed with alum (Table 1, pH 7.4 and pH 5.0). In the cases of any pH-sensitive compound, it was confirmed that mixing with alum produces no influence on the function of the pH-sensitive carrier.

In addition, the pH-sensitive carriers mixed with alum, in the cases of using any pH-sensitive compound, satisfied the formula (1) and (2), and, thus, surely had the effect of promoting the membrane-disruption function. Therefore, these pH-sensitive carriers are considered to induce cell-mediated immunity by being mixed with alum (Table 1).

[Table 1]

| | | | Cholic acid | Ursodeoxycholic acid | Chenodeoxycholic acid | Hyodeoxycholic acid | Glycodeoxycholic acid | Glycyrrhizinic acid |
|---|---|---|---|---|---|---|---|---|
| pH 7.4 Leakage (%) | $Lc'_{7.4}$ | pH-sensitive carrier + alum | 1.0 | 1.7 | 5.5 | 4.3 | 3.9 | 1.1 |
| | $Lc_{7.4}$ | pH-sensitive carrier | 0.1 | 0.8 | 3.8 | 1.5 | 0.8 | 0.4 |
| | $Lc_{7.4}$ | pH-sensitive compound alone | 0.6 | 0.8 | 1.1 | 0.6 | 1.6 | 0.8 |
| pH 5.0 Leakage (%) | $Lc'_{5.0}$ | pH-sensitive carrier + alum | 7.7 | 16.6 | 64.9 | 39.9 | 14.2 | 28.2 |
| | $Lc_{5.0}$ | pH-sensitive carrier | 8.1 | 13.1 | 64.1 | 43.9 | 11.7 | 30.7 |
| | $La_{5.0}$ | pH-sensitive compound alone | 1.9 | 2.6 | 3.1 | 2.8 | 5.9 | 3.0 |
| | $Lb_{5.0}$ | Amphipathic substance (DLPC) alone | 1.9 | 1.9 | 1.9 | 1.9 | 1.9 | 1.9 |
| Formula (1)* | $\Delta$ | pH-sensitive carrier + alum | 5.4 | 13.1 | 57.4 | 33.4 | 6.0 | 24.9 |
| | | pH-sensitive carrier | 6.7 | 10.5 | 58.3 | 40.2 | 6.6 | 28.1 |
| Formula (2)* | $\Delta'$ | pH-sensitive carrier + alum | 3.9 | 12.1 | 59.9 | 35.2 | 6.4 | 23.3 |
| | | pH-sensitive carrier | 4.3 | 8.6 | 59.1 | 39.2 | 3.9 | 25.8 |

*In the case of pH-sensitive carrier

$\Delta = (Lc_{5.0} - Lc_{7.4}) - (La_{5.0} - La_{7.4})$

$\Delta' = Lc_{5.0} - (La_{5.0} + Lb_{5.0})$

*In the case of pH-sensitive carrier + alum

$\Delta = (Lc'_{5.0} - Lc'_{7.4}) - (La_{5.0} - La_{7.4})$

$\Delta' = Lc'_{5.0} - (La_{5.0} + Lb_{5.0})$

(7-5) Influence of alum in pH-sensitive carriers including various amphipathic substances

In addition, influence of alum on the functions of pH-sensitive carriers including various amphipathic substances wax examined. As alum was used 10 $\mu$g of aluminum hydroxide, and complexes of 100 nmol each of various amphipathic substances and 160 nmol of deoxycholic acid were used as the pH-sensitive carrier (total amount: 100 $\mu$L). Like in (7-1), dispersions (solutions) of pH-sensitive carriers each composed of 100 nmol of predetermined amphipathic substance and 160 nmol of deoxycholic acid and alum were mixed, the resulting mixed solutions were incubated overnight, and part of each of the mixed solutions was put to the release test. The release test was carried out according to the above-described release test.

In the cases of the pH-sensitive carriers prepared using any amphipathic substance, the pH-sensitive carrier mixed with alum showed leakage values not less than those shown by the pH-sensitive carrier not mixed with alum (Table 2-2 and Table 2-3). Note that the leakage of deoxycholic acid (pH-sensitive compound) used alone was not more than 1% at pH 7.4, and approximately 15% at pH 5.0.

Besides, the pH-sensitive carriers mixed with alum, in the cases of using any amphipathic substance, satisfied the formula (1) and formula (2), and, thus, surely had the effect of promoting the membrane-disruption function. Therefore, these pH-sensitive carriers are considered to induce cell-mediated immunity by being mixed with alum (Table 2-2 and Table 2-3).

[Table 2-1]

| Table 2-1. In regard of items in Table 2-2 and Table 2-3 | | |
|---|---|---|
| pH 7.4 Leakage(%) | Lc'7.4 | pH-sensitive carrier + alum |
| | Lc7.4 | pH-sensitive carrier |
| | La7.4 | pH-sensitive compound alone |
| | Lb7.4 | Amphipathic substance alone |
| pH 5.0 Leakage(%) | Lc'5.0 | pH-sensitive carrier + alum |
| | Lc5.0 | pH-sensitive carrier |
| | La5.0 | pH-sensitive compound alone |
| | Lb5.0 | Amphipathic substance alone |
| Formula (1) $\Delta$ | pH-sensitive carrier + alum | $\Delta=(Lc'_{5.0}-Lc'_{7.4})-(Lc'_{5.0}-La_{7.4})$ |
| | pH-sensitive carrier | $\Delta=(Lc_{5.0}-Lc_{7.4})-(La_{5.0}-La_{7.4})$ |
| Formula (2) $\Delta'$ | pH-sensitive carrier + alum | $\Delta'=Lc'_{5.0}-(La_{5.0}+Lb_{5.0})$ |
| | pH-sensitive carrier | $\Delta'=Lc_{5.0}-(La_{5.0}+Lb_{5.0})$ |

[Table 2-2]

| | | DDPC | Tween 20 | Tween 40 | Tween 60 | Tween 80 | Peg (10) castor oil | Span80 | Glycerol monooleate |
|---|---|---|---|---|---|---|---|---|---|
| pH 7.4 Leakage (%) | Lc'7.4 | 51.7 | 67.3 | 15.8 | 7.6 | 8.2 | 28.7 | 18.9 | 69.6 |
| | Lc7.4 | 52.7 | 70.0 | 7.8 | 4.7 | 2.1 | 7.5 | 14.0 | 62.5 |
| | La7.4 | 0.2 | 0.2 | 0.2 | 0.2 | 0.2 | 0.2 | 0.2 | 0.2 |
| | Lb7.4 | 8.4 | 50.4 | 8.3 | 3.8 | 0.6 | 3.4 | 4.4 | 4.1 |
| pH 5.0 Leakage (%) | Lc'5.0 | 99.2 | 95.7 | 80.8 | 26. 9 | 28.0 | 50.4 | 39.5 | 95.2 |
| | Lc5.0 | 97.7 | 94.6 | 68.4 | 21.9 | 23.8 | 29.1 | 42.6 | 86.3 |
| | La5.0 | 14.6 | 14.6 | 14.6 | 14.6 | 14.6 | 14.6 | 14.6 | 14.6 |
| | Lb5.0 | 10.1 | 48.8 | 11.5 | 6.7 | 1.6 | 6.2 | 7.3 | 6.0 |

(continued)

| | | DDPC | Tween 20 | Tween 40 | Tween 60 | Tween 80 | Peg (10) castor oil | Span80 | Glycerol monooleate |
|---|---|---|---|---|---|---|---|---|---|
| Formula (1) Δ | pH-sensitive carrier + alum | 41.3 | 64.2 | 58.7 | 8.5 | 5.8 | 10.5 | 10.4 | 15.1 |
| | pH-sensitive carrier | 38.8 | 60.4 | 54.3 | 6.4 | 7.7 | 10.4 | 18.4 | 13.3 |
| Formula (2) Δ' | pH-sensitive carrier + alum | 74.5 | 32.3 | 54.7 | 5.6 | 11.8 | 29.6 | 17.6 | 74.6 |
| | pH-sensitive carrier | 73.0 | 31.2 | 42.3 | 0.6 | 7.6 | 8.3 | 20.7 | 65.7 |

[Table 2-3]

| | | Span40 | Span60 | Span65 | Span85 | Tocopherol | Glycyrrhizinic acid | Glycerol distearate | $\alpha,\alpha'$-dilaurin |
|---|---|---|---|---|---|---|---|---|---|
| pH 7.4 Leakage(%) | Lc'7.4 | 8.2 | 10.5 | 7.5 | 17.7 | 7.6 | 21.8 | 21.8 | 13.8 |
| | Lc7 .4 | 2.1 | 2.8 | 2.2 | 1.8 | 10.4 | 12.2 | 12.2 | 0.9 |
| | La7.4 | 0.2 | 0.2 | 0.2 | 0.2 | 0.2 | 0.2 | 0.2 | 0.2 |
| | Lb7.4 | 0.6 | 1.6 | 1.5 | 1.8 | 1.7 | 0.3 | 0.3 | 1.0 |
| pH 5.0 Leakage (%) | Lc'5.0 | 28.0 | 36.5 | 39.5 | 54.2 | 45.7 | 38.2 | 38.6 | 30.4 |
| | Lc5.0 | 23.8 | 36.5 | 29.8 | 47.5 | 52.8 | 31.5 | 31.5 | 17.2 |
| | La5.0 | 14.6 | 14.6 | 14.6 | 14.6 | 14.6 | 14.6 | 14.6 | 14.6 |
| | Lb5.0 | 1.6 | 4.3 | 4.2 | 2.0 | 1.2 | 4.0 | 4.0 | 0.8 |
| Formula (1) $\Delta$ | pH-sensitive carrier + alum | 5.8 | 13.0 | 18.9 | 23.7 | 25.2 | 2.1 | 2.5 | 3.0 |
| | pH-sensitive carrier | 7.7 | 20.7 | 14.5 | 32.9 | 29.5 | 5.0 | 5.0 | 2.7 |
| Formula (2) $\Delta'$ | pH-sensitive carrier + alum | 11.8 | 17.6 | 20.7 | 37.6 | 29.9 | 19.6 | 20.0 | 15.0 |
| | pH-sensitive carrier | 7.6 | 17.6 | 11.0 | 30.9 | 37.0 | 12.9 | 12.9 | 1.8 |

EP 3 305 320 B1

(8) Study of administration route

In general, many immune-responsible cells are present in the skin, and it is known that by inoculating vaccine into the skin, it is possible to accentuate causing of immunity and to reduce the amount of antigen. In view of this, induction of cell-mediated immunity in the case of administering the vaccine composition into the skin was confirmed.

Aluminum hydroxide was used as alum, while a complex of DLPC and deoxycholic acid (100 nmol of DLPC and 160 nmol of deoxycholic acid) was used as the pH-sensitive carrier. In the case of subcutaneous administration, the dose per animal was 100 µL, and 10 µg of alum, 80 µg of OVA, and the pH-sensitive carrier composed of 100 nmol of DLPC were administered. Besides, in the case of intracutaneous administration, the dose per animal was 20 µL, and 2 µg of alum, 16 µg of OVA, and the pH-sensitive carrier composed of 20 nmol of DLPC were administered. Specifically, for intracutaneous administration, the administration liquid used in subcutaneous administration was used, with the dose changed from 100 to 20 µL, to thereby prepare the above-described amount. Induction of cell-mediated immunity was evaluated according to the above-described ELIsot method.

As a result of the evaluation, joint use of alum and the pH-sensitive carrier in the intracutaneous administration led to formation of spots the number of which was greater than that by alum alone (FIGS. 11(C) and (D)). The vaccine composition was confirmed to induce cell-mediated immunity, even when administered intracutaneously. In addition, comparison of this result with the result obtained in subcutaneous administration showed that intracutaneous administration of the vaccine composition led to formation of a greater number of spots than in the case of subcutaneous administration of the vaccine composition, notwithstanding the small dose (FIGS. 11 (B) and (D)). It was made clear that the vaccine composition's effect of inducing cell-mediated immunity is enhanced by intracutaneous administration.

Subsequently, antigen specificity of the induced cell-mediated immunity was evaluated. The evaluation was carried out according to the above-described ELIspot method in which restimulation is not applied. As a result, the intracutaneous administration of the vaccine composition did not form spots (FIG. 11(H)). It was made clear that the cell-mediated immunity induced by intracutaneous administration of the vaccine composition is antigen-specific.

The present application is based on Japanese Patent Application No. 2015-112519 filed on June 2, 2015.

**Claims**

1. An adjuvant composition comprising:

   a pH-sensitive carrier containing
   at least one pH-sensitive compound selected from the group consisting of deoxycholic acid, cholic acid, ursodeoxycholic acid, chenodeoxycholic acid, hyodeoxycholic acid, glycodeoxycholic acid, glycyrrhizinic acid, glycyrrhetinic acid, and salts thereof, and
   at least one amphipathic substance selected from the group consisting of phosphatidylcholine having only an acyl group having 10 to 12 carbon atoms, polyoxyethylene sorbitan monofatty acid ester having an acyl group having 12 to 18 carbon atoms, sorbitan fatty acid ester having only an acyl group having 16 to 18 carbon atoms, glycerol monooleate, glycerol dilaurate, glycerol distearate, glycerol dioleate, polyoxyethylene castor oil, and α-tocopherol, and
   an aluminum-containing substance, wherein the aluminum-containing substance is contained in an amount of 1 to 1,000 µg, as aluminum metal, per 100 nmol of the amphipathic substance.

2. The adjuvant composition according to claim 1, wherein the aluminum-containing substance is at least one selected from the group consisting of aluminum hydroxide and aluminum phosphate.

3. The adjuvant composition according to claim 1 or 2, wherein the pH-sensitive compound is contained in a proportion of not less than 10 mol per 100 mol of the amphipathic substance.

4. A vaccine composition comprising:

   the adjuvant composition according to claim 1 to 3; and
   an antigen.

5. The vaccine composition according to claim 4, wherein a content of the antigen is 3.2 to 400 µg per 100 nmol of the amphipathic substance.

6. The vaccine composition according to any one of claims 4 or 5, wherein the antigen is a peptide or a protein.

7. A method of producing an adjuvant composition, comprising

a step of associating
at least one pH-sensitive compound selected from the group consisting of deoxycholic acid, cholic acid, ursodeoxycholic acid, chenodeoxycholic acid, hyodeoxycholic acid, glycodeoxycholic acid, glycyrrhizinic acid, glycyrrhetinic acid, and salts thereof,
at least one amphipathic substance selected from the group consisting of phosphatidylcholine having only an acyl group having 10 to 12 carbon atoms, polyoxyethylene sorbitan mono fatty acid ester having an acyl group having 12 to 18 carbon atoms, sorbitan fatty acid ester having only an acyl group having 16 to 18 carbon atoms, glycerol monooleate, glycerol dilaurate, glycerol distearate, glycerol dioleate, polyoxyethylene castor oil, and $\alpha$-tocopherol, and
an aluminum-containing substance with one another, wherein the aluminum-containing substance is contained in an amount of 1 to 1,000 $\mu$g, as aluminum metal, per 100 nmol of the amphipathic substance.

8. A method of producing a vaccine composition, comprising:

a step of associating
at least one pH-sensitive compound selected from the group consisting of deoxycholic acid, cholic acid, ursodeoxycholic acid, chenodeoxycholic acid, hyodeoxycholic acid, glycodeoxycholic acid, glycyrrhizinic acid, glycyrrhetinic acid, and salts thereof,
at least one amphipathic substance selected from the group consisting of phosphatidylcholine having only an acyl group having 10 to 12 carbon atoms, polyoxyethylene sorbitan monofatty acid ester having an acyl group having 12 to 18 carbon atoms, sorbitan fatty acid ester having only an acyl group having 16 to 18 carbon atoms, glycerol monooleate, glycerol dilaurate, glycerol distearate, glycerol dioleate, polyoxyethylene castor oil, and $\alpha$-tocopherol, and
an aluminum-containing substance with one another, wherein the aluminum-containing substance is contained in an amount of 1 to 1,000 $\mu$g, as aluminum metal, per 100 nmol of the amphipathic substance; and
a step of mixing an antigen with an associated body obtained by the association.

## Patentansprüche

1. Adjuvans-Zusammensetzung, umfassend:

einen pH-empfindlichen Träger, der umfasst mindestens eine pH-empfindliche Verbindung, die ausgewählt ist aus der Gruppe, bestehend aus Desoxycholsäure, Cholsäure, Ursodeoxycholsäure, Chenodeoxycholsäure, Hyodeoxycholsäure, Glycodeoxycholsäure, Glycyrrhizinsäure, Glycyrrhetinsäure und Salzen davon, und mindestens eine amphipathische Substanz, die ausgewählt ist aus der Gruppe, bestehend aus Phosphatidylcholin, das nur eine Acylgruppe mit 10 bis 12 Kohlenstoffatomen aufweist, Polyoxyethylen-Sorbitan-Monofettsäureester, der eine Acylgruppe mit 12 bis 18 Kohlenstoffatomen aufweist, Sorbitanfettsäureester, der nur eine Acylgruppe mit 16 bis 18 Kohlenstoffatomen aufweist, Glycerinmonooleat, Glycerindilaurat, Glycerindistearat, Glycerindioleat, Polyoxyethylenrizinusöl und $\alpha$-Tocopherol, und
eine aluminiumhaltige Substanz, wobei die aluminiumhaltige Substanz in einer Menge von 1 bis 1000 $\mu$g als Aluminiummetall pro 100 nmol der amphipathischen Substanz enthalten ist.

2. Adjuvans-Zusammensetzung nach Anspruch 1, wobei die aluminiumhaltige Substanz mindestens eine Substanz ist, die aus der Gruppe ausgewählt ist, die aus Aluminiumhydroxid und Aluminiumphosphat besteht.

3. Adjuvans-Zusammensetzung nach Anspruch 1 oder 2, wobei die pH-empfindliche Verbindung in einem Anteil von nicht weniger als 10 Mol pro 100 Mol der amphipathischen Substanz enthalten ist.

4. Impfstoffzusammensetzung, umfassend:

die Adjuvans-Zusammensetzung nach einem der Ansprüche 1 bis 3; und
ein Antigen.

5. Impfstoffzusammensetzung nach Anspruch 4, wobei ein Gehalt des Antigens 3,2 bis 400 $\mu$g pro 100 nmol der amphipathischen Substanz beträgt.

6. Impfstoffzusammensetzung nach einem der Ansprüche 4 oder 5, wobei das Antigen ein Peptid oder ein Protein ist.

7. Verfahren zur Herstellung einer Adjuvans-Zusammensetzung, umfassend

einen Schritt des Assoziierens
von mindestens einer pH-empfindlichen Verbindung, die ausgewählt ist aus der Gruppe, bestehend aus Desoxycholsäure, Cholsäure, Ursodeoxycholsäure, Chenodeoxycholsäure, Hyodeoxycholsäure, Glycodeoxycholsäure, Glycyrrhizinsäure, Glycyrrhetinsäure und Salzen davon,
von mindestens einer amphipathischen Substanz, die ausgewählt ist aus der Gruppe, bestehend aus Phosphatidylcholin, das nur eine Acylgruppe mit 10 bis 12 Kohlenstoffatomen aufweist, Polyoxyethylen-Sorbitan-Monofettsäureester, der eine Acylgruppe mit 12 bis 18 Kohlenstoffatomen aufweist, Sorbitanfettsäureester, der nur eine Acylgruppe mit 16 bis 18 Kohlenstoffatomen aufweist, Glycerinmonooleat, Glycerindilaurat, Glycerindistearat, Glycerindioleat, Polyoxyethylenrizinusöl und $\alpha$-Tocopherol, und
einer aluminiumhaltigen Substanz miteinander, wobei die aluminiumhaltige Substanz in einer Menge von 1 bis 1000 $\mu$g als Aluminiummetall pro 100 nmol der amphipathischen Substanz enthalten ist.

8. Verfahren zur Herstellung einer Adjuvans-Zusammensetzung, umfassend:

einen Schritt des Assoziierens
von mindestens einer pH-empfindlichen Verbindung, die ausgewählt ist aus der Gruppe, bestehend aus Desoxycholsäure, Cholsäure, Ursodeoxycholsäure, Chenodeoxycholsäure, Hyodeoxycholsäure, Glycodeoxycholsäure, Glycyrrhizinsäure, Glycyrrhetinsäure und Salzen davon,
von mindestens einer amphipathischen Substanz, die ausgewählt ist aus der Gruppe, bestehend aus Phosphatidylcholin, das nur eine Acylgruppe mit 10 bis 12 Kohlenstoffatomen aufweist, Polyoxyethylen-Sorbitan-Monofettsäureester, der eine Acylgruppe mit 12 bis 18 Kohlenstoffatomen aufweist, Sorbitanfettsäureester, der nur eine Acylgruppe mit 16 bis 18 Kohlenstoffatomen aufweist, Glycerinmonooleat, Glycerindilaurat, Glycerindistearat, Glycerindioleat, Polyoxyethylenrizinusöl und $\alpha$-Tocopherol, und
von einer aluminiumhaltigen Substanz miteinander, wobei die aluminiumhaltige Substanz in einer Menge von 1 bis 1000 $\mu$g als Aluminiummetall pro 100 nmol der amphipathischen Substanz enthalten ist; und
einen Schritt des Mischens eines Antigens mit einem assoziierten Körper, der durch die Assoziation erhalten wurde.

**Revendications**

1. Composition d'adjuvant comprenant :

un support sensible au pH contenant
au moins un composé sensible au pH sélectionné dans le groupe consistant en l'acide désoxycholique, l'acide cholique, l'acide ursodésoxycholique, l'acide chénodésoxycholique, l'acide hyodésoxycholique, l'acide glycodésoxycholique, l'acide glycyrrhizinique, l'acide glycyrrhétinique, et les sels de ceux-ci, et
au moins une substance amphipathique sélectionnée dans le groupe consistant en une phosphatidylcholine ayant seulement un groupe acyle ayant 10 à 12 atomes de carbone, un ester de sorbitane de mono-acide gras polyoxyéthyléné ayant un groupe acyle ayant 12 à 18 atomes de carbone, un ester de sorbitane d'acide gras ayant seulement un groupe acyle ayant 16 à 18 atomes de carbone, le monooléate de glycérol, le dilaurate de glycérol, le distéarate de glycérol, le dioléate de glycérol, l'huile de ricin polyoxyéthylénée, et l'a-tocophérol, et
une substance contenant de l'aluminium, dans laquelle la substance contenant de l'aluminium est contenue en une quantité de 1 à 1000 $\mu$g, en tant qu'aluminium métallique, pour 100 nmoles de la substance amphipathique.

2. Composition d'adjuvant selon la revendication 1, dans laquelle la substance contenant de l'aluminium est au moins une sélectionnée dans le groupe consistant en l'hydroxyde d'aluminium et le phosphate d'aluminium.

3. Composition d'adjuvant selon la revendication 1 ou 2, dans laquelle le composé sensible au pH est contenu dans une proportion de pas moins de 10 moles pour 100 moles de la substance amphipathique.

4. Composition de vaccin comprenant :

la composition d'adjuvant selon les revendications 1 à 3 ; et

un antigène.

5. Composition de vaccin selon la revendication 4, dans laquelle une teneur de l'antigène est de 3,2 à 400 μg pour 100 nmoles de la substance amphipathique.

6. Composition de vaccin selon l'une quelconque des revendications 4 ou 5, dans laquelle l'antigène est un peptide ou une protéine.

7. Procédé de production d'une composition d'adjuvant, comprenant

une étape consistant à associer
au moins un composé sensible au pH sélectionné dans le groupe consistant en l'acide désoxycholique, l'acide cholique, l'acide ursodésoxycholique, l'acide chénodésoxycholique, l'acide hyodésoxycholique, l'acide glycodésoxycholique, l'acide glycyrrhizinique, l'acide glycyrrhétinique, et les sels de ceux-ci,
au moins une substance amphipathique sélectionnée dans le groupe consistant en une phosphatidylcholine ayant seulement un groupe acyle ayant 10 à 12 atomes de carbone, un ester de sorbitane de mono-acide gras polyoxyéthyléné ayant un groupe acyle ayant 12 à 18 atomes de carbone, un ester de sorbitane d'acide gras ayant seulement un groupe acyle ayant 16 à 18 atomes de carbone, le monooléate de glycérol, le dilaurate de glycérol, le distéarate de glycérol, le dioléate de glycérol, l'huile de ricin polyoxyéthylénée, et l'a-tocophérol, et
une substance contenant de l'aluminium les uns aux autres, dans lequel la substance contenant de l'aluminium est contenue en une quantité de 1 à 1000 μg, en tant qu'aluminium métallique, pour 100 nmoles de la substance amphipathique.

8. Procédé de production d'une composition de vaccin, comprenant :

une étape consistant à associer
au moins un composé sensible au pH sélectionné dans le groupe consistant en l'acide désoxycholique, l'acide cholique, l'acide ursodésoxycholique, l'acide chénodésoxycholique, l'acide hyodésoxycholique, l'acide glycodésoxycholique, l'acide glycyrrhizinique, l'acide glycyrrhétinique, et les sels de ceux-ci,
au moins une substance amphipathique sélectionnée dans le groupe consistant en une phosphatidylcholine ayant seulement un groupe acyle ayant 10 à 12 atomes de carbone, un ester de sorbitane de mono-acide gras polyoxyéthyléné ayant un groupe acyle ayant 12 à 18 atomes de carbone, un ester de sorbitane d'acide gras ayant seulement un groupe acyle ayant 16 à 18 atomes de carbone, le monooléate de glycérol, le dilaurate de glycérol, le distéarate de glycérol, le dioléate de glycérol, l'huile de ricin polyoxyéthylénée, et l'a-tocophérol, et
une substance contenant de l'aluminium les uns aux autres, dans lequel la substance contenant de l'aluminium est contenue en une quantité de 1 à 1000 μg, en tant qu'aluminium métallique, pour 100 nmoles de la substance amphipathique ; et
une étape consistant à mélanger un antigène avec un corps associé obtenu par l'association.

# FIG. 1

# F I G . 2

| | ANTIGEN ALONE | ANTIGEN + TOCOPHEROL | ANTIGEN + TOCOPHEROL ACETATE | ANTIGEN + Alum |
|---|---|---|---|---|
| pH-SENSITIVE CARRIER ABSENT | (A) | (B) | (C) | (D) |
| INDUCTION OF CELL-MEDIATED IMMUNITY | × | × | × | × |
| pH-SENSITIVE CARRIER PRESENT | | (E) | (F) | (G) |
| INDUCTION OF CELL-MEDIATED IMMUNITY | | × | × | ○ |

# F I G . 3

| | (A) ANTIGEN + Alum | (B) ANTIGEN + Alum + pH-SENSITIVE CARRIER | (C) ANTIGEN + CpG | (D) ANTIGEN + Alum + pH-SENSITIVE CARRIER (EVALUATION OF ANTIGEN SPECIFICITY) |
|---|---|---|---|---|
| VARIOUS EVALUATION SAMPLES | | | | |
| INDUCTION OF CELL-MEDIATED IMMUNITY | × | ○ | ○ | × |

# F I G . 4

| VARIOUS EVALUATION SAMPLES | (A) ANTIGEN ALONE | (B) ANTIGEN + Alum 10μg | | |
|---|---|---|---|---|
| INDUCTION OF CELL-MEDIATED IMMUNITY | × | × | | |
| VARIOUS EVALUATION SAMPLES | (C) ANTIGEN + Alum 100 μg + pH-SENSITIVE CARRIER | (D) ANTIGEN + Alum 10μg + pH-SENSITIVE CARRIER | (E) ANTIGEN + Alum 1μg + pH-SENSITIVE CARRIER | (F) ANTIGEN + Alum 0.1μg + pH-SENSITIVE CARRIER |
| INDUCTION OF CELL-MEDIATED IMMUNITY | ○ | ○ | ○ | × |

# F I G . 5

| VARIOUS EVALUATION SAMPLES | (A) ANTIGEN ALONE | (B) ANTIGEN + Alum 10μg | | |
|---|---|---|---|---|
| INDUCTION OF CELL-MEDIATED IMMUNITY | × | × | | |
| VARIOUS EVALUATION SAMPLES | (C) ANTIGEN + Alum 10μg + pH-SENSITIVE CARRIER | (D) ANTIGEN + Alum 10μg + 10-FOLD DILUTED pH-SENSITIVE CARRIER | (E) ANTIGEN + Alum 10μg + 100-FOLD DILUTED pH-SENSITIVE CARRIER | (F) ANTIGEN + Alum 10μg + 1000-FOLD DILUTED pH-SENSITIVE CARRIER |
| INDUCTION OF CELL-MEDIATED IMMUNITY | ○ | ○ | ○ | △ |

# FIG. 6

EVERY ADMINISTRATION GROUP CONTAINS OVA PROTEIN AS ANTIGEN

# FIG. 7

# F I G . 8

(A)

(B)

LEAKAGE AT pH 5.0

AMOUNT OF alum PER 100 nmol OF DLPC
($\mu$g)

# F I G . 9

# F I G . 1 0

(A)

AMOUNT OF DEOXYCHOLIC ACID PER 100 nmol OF DLPC
(nmol)

(B)

ENLARGED DIAGRAM OF (A)

AMOUNT OF DEOXYCHOLIC ACID PER 100 nmol OF DLPC
(nmol)

# FIG.11

| | SUBCUTANEOUS ADMINISTRATION 100μL/head | | INTRACUTANEOUS ADMINISTRATION 20μL/head | |
|---|---|---|---|---|
| | ANTIGEN + Alum (Alum ALONE) | ANTIGEN + Alum + pH-SENSITIVE CARRIER | ANTIGEN + Alum (Alum ALONE) | ANTIGEN + Alum + pH-SENSITIVE CARRIER |
| EVALUATION BY ELIspot METHOD | (A) | (B) | (C) | (D) |
| INDUCTION OF CELL-MEDIATED IMMUNITY | × | ○ | × | ○ |
| EVALUATION BY ELIspot METHOD ANTIGEN SPECIFICITY EVALUATED | (E) | (F) | (G) | (H) |
| INDUCTION OF CELL-MEDIATED IMMUNITY | × | × | × | × |

**EP 3 305 320 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013180253 A

- JP 2015112519 A

**Non-patent literature cited in the description**

- *Bioconjug. Chem.,* February 2009, vol. 20 (2), 241-248
- *Immune Netw.,* October 2013, vol. 13 (5), 177-183
- *Immunology and Cell Biology,* 2004, vol. 82, 497-505
- **K. KONO et al.** *Bioconjugate Chem.,* 2008, vol. 19, 1040-1048
- **K. KONO et al.** *Biomaterials,* 2008, vol. 29, 4029-4036